(19) Europäisches Patentamt
European Patent Office
Office européen des brevets

(11) EP 2 354 232 A1

(12) # EUROPEAN PATENT APPLICATION

(43) Date of publication:
**10.08.2011 Bulletin 2011/32**

(51) Int Cl.:
***C12N 15/82*** (2006.01) ***C12N 9/06*** (2006.01)

(21) Application number: **10290058.6**

(22) Date of filing: **08.02.2010**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(71) Applicant: **Genoplante-Valor**
**75015 Paris (FR)**

(72) Inventors:
- **Salse, Jérôme**
  **63610 Valbeleix (FR)**
- **Quraishi, Umar Masood**
  **63100 Clermont-Ferrand (FR)**
- **Pont, Caroline**
  **63610 Valbeleix (FR)**
- **Murat, Florent**
  **63000 Clermont-Ferrand (FR)**
- **Le Gouis, Jacques**
  **63320 Champeix (FR)**
- **Lafarge, Stéphane**
  **63670 La Roche Blanche (FR)**

(74) Representative: **Vialle-Presles, Marie José et al**
**Cabinet ORES**
**36, rue de St Pétersbourg**
**75008 Paris (FR)**

(54) **Improvement of the grain filling of wheat through the modulation of NADH-glutamate synthase activity**

(57) The invention relates to a method for increasing the grain filling of a wheat plant, wherein said method comprises overexpressing in said plant a wheat NADH-dependent glutamate synthase, in order to increase the grain weight and/or the grain protein content.

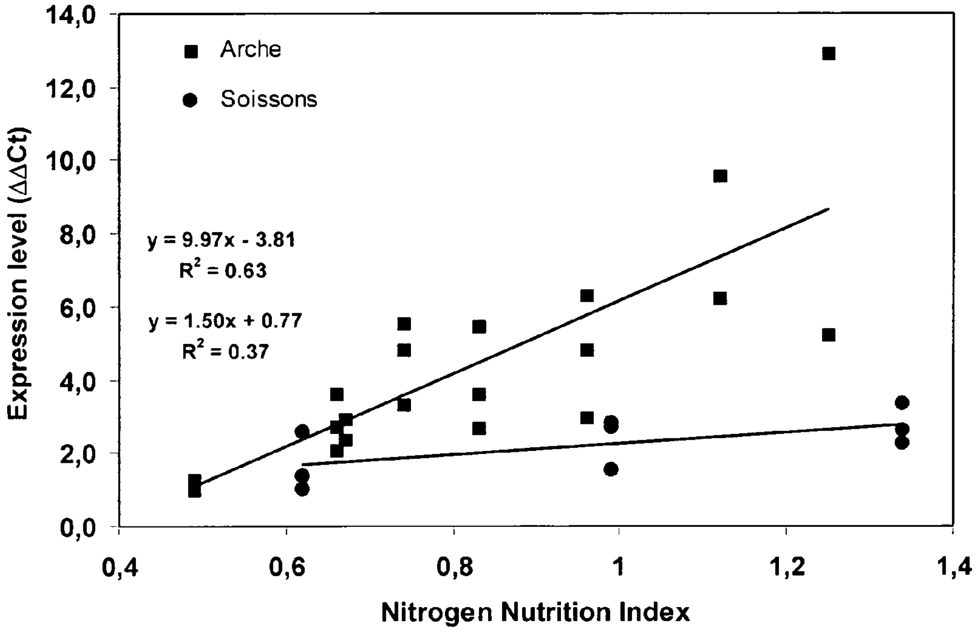


**Figure 1**

EP 2 354 232 A1

## Description

The present invention relates to methods for controlling yield of wheat through the modulation of NADH-dependent glutamate synthase (NADH-GoGAT) activity.

High grain yield with adequate protein content is an important goal in crop improvement especially in bread wheat (*Triticum aestivum L.*). Unfortunately, it has been shown in various cereals including wheat that these two traits are genetically negatively correlated either in extensive North American farming or in intensive farming in Europe (Simmonds 1995; Oury *et al.,* 2003). This correlation can be broken down by adequate nitrogen (N) supply late in the plant development (Krapp *et al.,* 2005; Laperche *et al.,* 2006). Nitrogen fertilizers are used as an important agronomic tool to improve output quantity as well as quality in all cultivated crops. However, the current agricultural and economic environment concerns impose farmers to constantly optimize the application of nitrogen fertilizers in order to avoid pollution by nitrates and preserve their economic margin.

Therefore, the selection for cereal cultivars that absorb and metabolize nitrogen in the most efficient way for grain or silage production is becoming increasingly important. Such improved crops would make a better use of nitrogen fertilizer supplies as they would produce higher yields with better protein content. This might be achieved, at least in part, through a better understanding of nitrogen metabolism and its regulation, and by identifying target genes to monitor nitrogen uptake by either direct gene transfer or marker-assisted breeding. Either directly for the grain protein content or indirectly for the photosynthetic production in plant, nitrogen uptake is an essential element in crop improvement.

Some genetic variability in nitrogen use efficiency (NUE) and its components, namely nitrogen uptake and nitrogen utilization, has been reported in rice (Borrell *et al.,* 1998) and wheat (Le *Gouis et al.,* 2000). Further, various QTL (Quantitative Trait Loci) analyses for NUE have been performed during the last decades for barley (Kjaer and Jensen, 1995), maize (Agrama *et al.,* 1999; Bertin and Gallais, 2001; *Hirel et al.,* 2001) rice *(Obara et al.,* 2001; Lian *et al.,* 2005), and wheat (An *et al.* 2006; Laperche *et al.,* 2007; Habash *et al.,* 2007; Fontaine *et al.,* 2009), and for *Arabidopsis thaliana* (Rauh *et al.,* 2002; *Loudet et al.,* 2003). Major enzyme coding genes have been cloned and shown to drive nitrogen economy in plants (for review Miin and Habash, 2002; Bernard and Habash, 2009).

The glutamine synthetase (GS; E.C.6.3.1.2) is the first key enzyme for nitrogen metabolism, as it catalyses the assimilation of all inorganic nitrogen incorporated into organic compounds, such as proteins and nucleic acids. This reaction is coupled to the formation of glutamate by glutamate synthase (GoGAT) as part of the GS/GoGAT cycle.

In rice, two GoGAT types have been identified: a Ferredoxin (Fd)-dependent GoGAT (E.C. 1.4.7.1) and a NADH-dependent GoGAT (E.C. 1.4.1.14). Fd-GoGAT is known to be involved in photorespiration (Ireland and Lea, 1999). NADH-GoGAT is active in developing organs, such as unexpanded non-green leaves and developing grains (Yamaya *et al.,* 1992).

NADH-GoGAT catalyzes the reductive transfer of amide group of glutamine to 2-oxoglutarate to form two glutamate molecule (Krapp *et al.,* 2005):

$$2\ \text{L-glutamate} + NAD^+ \rightleftharpoons \text{L-glutamine} + \text{2-oxoglutarate} + NADH + H^+$$

It is hypothesized that NADH-GoGAT is probably involved in the utilization of remobilized nitrogen, since this protein is located in the specific cell types which are important for solute transport from the phloem and xylem elements (Hayakawa *et al.,* 1994). Yamaya *et al.* (2002) reported that, in rice, GoGAT enhances the grain filling suggesting that it is one of the potential candidate genes for NUE determinant.

Further, although Ferredoxin-GoGAT plays a critical part in the re-assimilation of ammonium released by glycine decarboxylase during photorespiration, NADH-GoGAT is involved in the assimilation of ammonium from both primary and secondary sources during nitrogen remobilization (Lea and Miin, 2003).

Genes coding for these two key enzymes involved in the $NH_4$ assimilation (*GS* and *NADH-GoGAT*) have been cloned in monocots such as rice (Tabuchi *et al.,* 2007 for both GS and *NADH-GoGAT;* Cai *et al.,* 2009 for GS), wheat (Caputo *et al.,* 2009 showing a physiological role of GS in the modulation of amino acids export levels in wheat) and maize (Valadier *et al.,* 2008 for both GS and *NADH-GoGAT*)*;* and eudicots such as *Arabidopsis* (Ishiyama *et al.,* 2004 for *GS;* Potel *et al.,* 2009 for *NADH-GoGAT*), Brassicaceae (Ochs *et al.,* 1999 for GS) and *Medicago* (Lima *et al.,* 2006 for GS).

Bread wheat is a hexaploid species with three diploid genomes named A, B and D; each genome consisting of seven pairs of chromosomes. The interactions between these 3 genomes are still unclear. Several putative *NADH-GoGAT* expressed sequences tags (ESTs), homolog to *NADH-GoGAT* ESTs in rice, have been found in bread wheat. However, until now, the functional ortholog of rice *NADH-GoGAT* has not been cloned in bread wheat.

The inventors have now found, in bread wheat, a *NADH-GoGAT* gene which plays a major role in driving NUE. This gene is located on chromosome 3B.

The inventors have also found that the wheat NADH-GoGAT proteins playing a major role in driving NUE show at least 95% identity between them and that such a wheat NADH-GoGAT protein has a percent identity inferior or equal to 91 % with the rice NADH-GoGAT, whose the amino acid sequence is available in GENBANK database under accession number GI:115439209 (and herein reproduced as SEQ ID NO: 6).

Accordingly, the present invention provides a method for improving the grain filling of a wheat plant, wherein said method comprises overexpressing in said plant a NADH-dependent glutamate synthase (NADH-GoGAT) having at least 95% identity, or by order of increasing preference at least 96%, 97%, 98% or 99% identity, with the polypeptide of sequence SEQ ID NO: 1 (corresponding to the NADH-GoGAT amino acid sequence of the bread wheat cultivar *Chinese Spring*).

Unless otherwise specified, the percents of identity between two sequences which are mentioned herein are calculated from an alignment of the two sequences over their whole length.

The term "overexpressing" a NADH-dependent glutamate synthase (NADH-GoGAT) in a wheat plant, herein refers to artificially increasing the quantity of said NADH-GoGAT produced in said plant compared to a reference plant.

The terms "wheat plant" and "wheat plant cell" as used herein, include any plant or plant cell of the genus *Triticum,* preferably of the species *Triticum aestivum L.* (bread wheat).

According to a preferred embodiment of the invention the grain filling is improved by increasing the grain weight and/or the grain protein content.

According to another preferred embodiment of the invention said NADH-GoGAT is encoded by a nucleotide sequence selected from the group consisting of SEQ ID NO: 2, SEQ ID NO: 3 and SEQ ID NO: 4. These nucleotide sequences correspond respectively to the genomic DNA sequences encoding the NADH-GoGAT protein of the bread wheat cultivars *Chinese Spring, Arche* and *Récital.*

According to another preferred embodiment of the invention said NADH-GoGAT is encoded by the nucleotide sequence SEQ ID NO: 5, corresponding to the coding DNA sequence (CDS) of *Chinese Spring NADH-GoGAT* gene.

A preferred method for overexpressing a NADH-GoGAT comprises introducing into the genome of said plant a DNA construct comprising a nucleotide sequence encoding said NADH-GoGAT, placed under control of a promoter.

The instant invention also provides means for carrying out said overexpression.

This includes, in particular, recombinant DNA constructs for expressing a NADH-GoGAT in a host-cell, or a host organism, in particular a wheat plant cell or a wheat plant. These DNA constructs can be obtained and introduced in said host cell or organism by the well-known techniques of recombinant DNA and genetic engineering.

Recombinant DNA constructs of the invention include in particular expression cassettes, comprising a polynucleotide encoding a NADH-GoGAT as defined above, under control of a heterologous promoter functional in plant cell.

The expression cassette of the invention may comprise a polynucleotide encoding at least two identical or different NADH-GoGAT as defined above.

The heterologous promoter of the invention is any promoter functional in a wheat plant cell, *i.e.*, capable of directing transcription of a polynucleotide encoding a NADH-GoGAT as defined above, in a wheat plant cell. The choice of the more appropriate promoter may depend in particular on the organ(s) or tissue(s) targeted for expression, and on the type of expression (*i.e.* constitutive or inducible) that one wishes to obtain.

A large choice of promoters suitable for expression of heterologous genes in wheat plants is available in the art. They can be obtained for instance from plants, plant viruses, or bacteria such as *Agrobacterium.* They include constitutive promoters, i.e. promoters which are active in most tissues and cells and under most environmental conditions, tissue or cell specific promoters which are active only or mainly in certain tissues or certain cell types, and inducible promoters that are activated by physical or chemical stimuli.

Non-limitative examples of constitutive promoters that are commonly used are the cauliflower mosaic virus (CaMV) 35S promoter, the nopaline synthase (Nos) promoter, the Cassava vein Mosaic Virus (CsVMV) promoter (Verdaguer *et al.,* 1996), the rice actin promoter followed by the rice actin intron (RAP-RAI) contained in the plasmid pAct1-F4 (McElroy *et al. ,* 1991).

Non-limitative examples of organ or tissue specific promoters that can be used in the present invention include for instance High Molecular Weight (HMW) promoter which is kernel specific (Thomas and Flavell, 1990), or the leaf specific promoters as pPEPc promoter (Jeanneau *et al.,* 2002), or the Rubisco small subunit promoter (rbcS) *(Katayama et al.,* 2000) which is specific of the bundle-sheath.

Inducible promoters include for instance drought stress responsive promoters, such as the rd29A promoter which comprises a dehydration-responsive element (Kasuga *et al.,* 1999; Narusaka *et al.,* 2003), or the senescence specific SAG12 promoter (Noh and Amasino, 1999).

The expression cassettes generally also include a transcriptional terminator, such as the 35S transcriptional terminator or Nos terminator (Depicker *et al.,* 1982). They may also include other regulatory sequences, such as transcription enhancer sequences.

Recombinant DNA constructs of the invention also include recombinant vectors containing an expression cassette comprising a polynucleotide encoding a NADH-GoGAT as defined above, under transcriptional control of a suitable promoter. Said expression cassette may be a recombinant expression cassette of the invention, or a cassette wherein the polynucleotide encoding a NADH-GoGAT is under control of its endogenous promoter.

A recombinant vector of the invention may include at least two polynucleotides encoding two identical or different NADH-GoGAT as defined above.

Recombinant vectors of the invention may also include other sequences of interest, such as, for instance, one or more marker genes, which allow for selection of transformed hosts.

Advantageously, the selectable marker gene is comprised between two Ds *(Dissociation)* elements (*i.e.*, transposons) in order for its removal at a later stage by interacting with the Ac (Activator) transposase. This elimination system is known from one skilled in the art. By way of example, it has been described in Goldsbrough *et al.* (1993).

The selection of suitable vectors and the methods for inserting DNA constructs therein are well known to persons of ordinary skill in the art. The choice of the vector depends on the intended host and on the intended method of transformation of said host.

A variety of techniques for genetic transformation of wheat plant cells or wheat plants are available in the art. By way of non-limitative examples, one can mention methods of direct transfer of genes such as direct micro-injection into plant embryoids, vacuum infiltration (Bechtold *et al.* 1993) or electroporation (Chupeau *et al.,* 1989), or the bombardment by gun of particules covered with the plasmidic DNA of interest *(Fromm et al.,* 1990; Finer *et al.,* 1992). *Agrobacterium* mediated transformation methods may also be used such as *Agrobacterium tumefaciens,* in particular according to the method described in the article by An *et al.* (1986), or *Agrobacterium rhizogenes,* in particular according to the method described in the article by Guerche *et al.,* (1987). According to a particular embodiment, it is possible to use the method described by Ishida *et al.* (1996) for the transformation of maize. According to another embodiment, the wheat is transformed according to the method described in International Application WO 00/63398.

The invention also comprises host cells containing a recombinant DNA construct of the invention. These host cells can be prokaryotic cells or eukaryotic cells, in particular plant cells, and preferably wheat plant cells.

The invention also provides a method for producing a transgenic wheat plant having an improved grain filling. Said method comprises transforming a wheat plant cell by a DNA construct of the invention and regenerating from said wheat plant cell a transgenic wheat plant overexpressing a NADH-GoGAT as defined above.

According to a preferred embodiment of the method of the invention, it comprises transforming a wheat plant cell by a recombinant vector of the invention comprising a polynucleotide encoding a NADH-GoGAT as defined above, and regenerating from said wheat plant cell a transgenic wheat plant overexpressing a NADH-GoGAT as defined above.

The invention also comprises wheat plants genetically transformed by a recombinant DNA construct of the invention, and overexpressing a NADH-GoGAT as defined above. In said transgenic plants a DNA construct of the invention is comprised in a transgene stably integrated in the plant genome, so that it is passed onto successive plant generations. Thus the transgenic wheat plants of the invention include not only the plants resulting from the initial transgenesis, but also their descendants, as far as they contain a recombinant DNA construct of the invention. The overexpression of a NADH-GoGAT as defined above in said plants provides them an improved grain filling, when compared with a plant devoid of said transgene(s).

The invention also comprises a transgenic wheat plant, obtainable by a method of the invention, overexpressing a NADG-GoGAT as defined above.

The invention further comprises a transgenic wheat plant or an isolated organ or tissue thereof comprising, stably integrated in its genome, a recombinant expression cassette comprising a polynucleotide encoding a NADH-GoGAT as defined above.

Accordingly, the invention also encompasses isolated organs or tissues of said transgenic wheat plant (such as seeds, leafs, flowers, roots, stems, ears) containing a recombinant expression cassette of the invention.

The present invention also provides an isolated polynucleotide chosen from the group consisting of:

a) a polynucleotide encoding a wheat NADH-GoGAT involved in Nitrogen Use Efficiency, which polypeptide has at least 95%, or by order of increasing preference at least 96%, 97% 98% or 99% identity, with the polypeptide of sequence SEQ ID NO: 1;
b) a polynucleotide complementary to the polynucleotide a);
c) a polynucleotide capable of hybridizing selectively, under stringent conditions, with the polynucleotide a) or the polynucleotide b).

According to a preferred embodiment, the polynucleotide encoding a wheat NADH-GoGAT is selected from the group consisting of sequences SEQ ID NO: 2, 3 and 4, which respectively correspond to the allelic form of the *NADH-GoGAT* gene of the bread wheat cultivars *Chinese Spring, Arche* and *Recital* and the sequence SEQ ID NO: 5, which corresponds to the CDS of the *Chinese Spring NADH-GoGAT* gene.

Stringent hybridization conditions, for a given nucleotide, can be identified by those skilled in the art according to the size and the base composition of the polynucleotide concerned, and also according to the composition of the hybridization mixture (in particular pH and ionic strength). Generally, stringent conditions, for a polynucleotide of given size and given sequence, are obtained by carrying out procedures at a temperature approximately 5°C to 10°C below the melting temperature (Tm) of the hybrid formed, in the same reaction mixture, by this polynucleotide and the polynucleotide complementary thereto.

A "polynucleotide capable of hybridizing selectively with a polynucleotide a) or b) in accordance with the invention" is here defined as any polynucleotide which, when it is hybridized under stringent conditions with a wheat nucleic acid library (in particular a genomic DNA or cDNA library), produces a detectable hybridization signal (*i.e.* at least twice as great, preferably at least five times as great, as the background noise) with said polynucleotide, but produces no detectable signal with other sequences of said library, and in particular with sequences encoding other proteins of the GoGAT family.

A subject of the present invention is also polynucleotide probes or amplification primers obtained from polynucleotides a) or b) in accordance with the invention or fragments thereof.

The present invention also encompasses any polynucleotide encoding a wheat NADH-GoGAT involved in Nitrogen Use Efficiency (NUE) and which can be obtained from a plant genomic DNA or cDNA library by screening said library with probes or primers in accordance with the invention. This includes in particular other alleles of the wheat *NADH-GoGAT* gene, and in particular other alleles capable of conferring an improved NUE and/or grain filling.

By way of example, one can also use the following pair of primers:

- TTAGTGGCAAATGGGCTTCG (SEQ ID NO: 7) and CGCCACAGCAACATCTCTACC (SEQ ID NO: 8);
- CAGCTGCAGAGATTCGTCCTG (SEQ ID NO: 9) and TGTTATCCAAAGCCATGTCAAGG (SEQ ID NO: 10);
- TGGAATGGCAGCAGAAAGGT (SEQ ID NO: 11) and TCGCATCCATGATCACCAATT (SEQ ID NO: 12);
- GCACCATTTCTGTACACTCGTTG (SEQ ID NO: 13) and ATCTTCCCATCAGTCTGCAAGC (SEQ ID NO: 14);
- TTCAAGAGCTTAACAAGGCGTG (SEQ ID NO: 15) and CACTTGCAGGTTCAACCTCATC (SEQ ID NO: 16);
- TGGGAAATGATGCACCCCTA (SEQ ID NO: 17) and CTTGTGCAAACATCTGCTTGAAG (SEQ ID NO: 18); and
- AATTCTGGAAGGAAGGGCTTG (SEQ ID NO: 19) and TTTGTATCCCTCGCGTATAGCTT (SEQ ID NO: 20);

preferably SEQ ID NO: 19 and 20, to amplify a fragment of a wheat mRNA of a *NADH-GoGAT* gene.

The invention also provides means for identifying and selecting wheat plants which have an improved grain filling compared to a reference wheat plant.

The invention thus provides a method for identifying an allele of a wheat *NADH-GoGAT* gene associated with a given phenotype of grain filling, wherein said method comprises isolating a nucleic acid fragment comprising said *NADH-GoGAT* gene or a portion thereof from at least one wheat plant expressing said phenotype, and sequencing said fragment.

The invention further provides a method for identifying polymorphisms associated with grain filling, in an *NADH-GoGAT* gene, wherein said method comprises identifying, as described above, at least two different alleles of said *NADH-GoGAT* gene associated with different phenotypes of grain filling, and comparing the sequences of said alleles.

Based on the *GoGAT* allele sequences characterised in *Chinese Spring, Arche* and *Récital* genotypes, the inventors have identified 23 DNA sequence variations (18 Single Nucleotide Polymorphisms (SNPs) and 5 Insertion / Deletion (InDels)) shown in Table 1 below, that can be used in Marker Assisted Selection (MAS) breeding programs for improving the grain filling of a wheat plant NUE improvement for instance.

Table 1: Detailed information regarding 18 SNP and 5 InDels identified between *Chinese Spring, Récital* and *Arche* genotypes. SNP and InDels coordinates are based on the *Chinese Spring* (CS) allele (SEO ID NO: 2).

| Base Coordinate (CS allele) | *Chinese Spring* | *Arche* | *Récital* |
|---|---|---|---|
| #2545 | A | A | G |
| #2663 | A | G | A |
| #2737 | G | G | A |
| #2871 | T | T | C |
| #2892 | G | G | T |
| #3010 | G | G | A |
| #3039 | A | A | G |
| #3512 | G | A | G |

(continued)

| Base Coordinate (CS allele) | *Chinese Spring* | *Arche* | *Récital* |
|---|---|---|---|
| #4752 | G | G | C |
| #5426 | C | C | T |
| #5452 | x | x | A |
| #5509 | G | G | A |
| #5681 | G | A | A |
| #6420 | x | G | G |
| #6916 | G | x | G |
| #8253 | A | G | G |
| #8882 | G | x | A |
| #8943 | A | G | G |
| #9404 | A | A | x |
| 9489 | T | A | A |
| #9541 | A | G | G |
| #9566 | T | G | G |
| #9592 | G | x | x |
| A, C, G and T represent the 4 nucleotide bases, repectively adenine, cytosine, guanine and thymine. "x" represents a deletion. | | | |

Once a polymorphism has been identified, reagents and kits allowing the routine detection of said polymorphism can be designed. Commonly used reagents are nucleic acid probes, or restriction enzymes, or PCR primers, or combinations thereof. The choice of a reagent or of a combination of reagents depends of the nature of the polymorphism.

Preferred kits and reagents are those comprising a set of primers allowing specific PCR amplification of a DNA segment spanning the polymorphic locus. For microsatellites and insertion/deletion polymorphisms, PCR primers may be sufficient, since the allelic forms of the polymorphism may be differentiated by the size of the amplification product. In the case of single nucleotide polymorphisms (SNP), one will generally also use a restriction enzyme, which allows the differentiation of allelic forms by the presence or size of restriction fragments.

For these purposes, it is possible to use a nucleic acid encoding a NADH-GoGAT as defined above, or a fragment thereof, as a probe or a target for amplification, for selecting wheat plants naturally overexpressing a NADH-GoGAT as defined above, and therefore exhibiting an improved grain filling. Preferably, the amplified fragment has a length of about 500 pb, more preferably, of about 500 to 1000 pb.

The Inventors also disclose a method for inhibiting in a wheat plant a NADH-dependent glutamate synthase (NADH-GoGAT) having at least 95% identity, or by order of increasing preference at least 96%, 97%, 98% or 99% identity, with the polypeptide of sequence SEQ ID NO: 1 as defined above.

The inhibition of a NADH-GoGAT protein can be obtained either by abolishing, blocking or decreasing its function (*i.e.* catalyzing the reductive transfer of amide group of glutamine to 2-oxoglutarate to form two glutamate molecule), or advantageously, by preventing or down-regulating the expression of its gene.

By way of example, inhibition of said NADH-GoGAT protein can be obtained by mutagenesis of the corresponding gene or of its promoter, and selection of the mutants having partially or totally lost the NADH-GoGAT protein activity. For instance, a mutation within the coding sequence can induce, depending on the nature of the mutation, the expression of an inactive protein, or of a protein with impaired activity; in the same way, a mutation within the promoter sequence can induce a lack of expression of said NADH-GoGAT protein, or decrease thereof.

Mutagenesis can be performed for instance by targeted deletion of the *NADH-GoGAT* coding sequence or promoter, or of a portion thereof, or by targeted insertion of an exogenous sequence within said coding sequence or said promoter. It can also be performed by random chemical or physical mutagenesis, followed by screening of the mutants within the *NADH-GoGAT* gene. Methods for high throughput mutagenesis and screening are available in the art. By way of example, one can mention TILLING (Targeting Induced Local Lesions IN Genomes, described by McCallum *et al.,* 2000).

Advantageously, the inhibition of said NADH-GoGAT protein is obtained by silencing of the corresponding gene. Methods for gene silencing in plants are known in themselves in the art. For instance, one can mention by antisense inhibition or co-suppression, as described by way of example in U.S. Patents 5,190,065 and 5,283,323. It is also possible to use ribozymes targeting the mRNA of said NADH-GoGAT protein.

Preferred methods are those wherein post transcriptional gene silencing is induced by means of RNA interference (RNAi) targeting the *NADH-GoGAT* gene to be silenced. Various methods and DNA constructs for delivery of RNAi are available in the art (for review, cf. *Watson et al.,* 2005). Typically, DNA constructs for delivering RNAi in a plant include at least a fragment of 300 bp or more (generally 300-800 bp, although shorter sequences may sometime induce efficient silencing) of the cDNA of the target gene, under transcriptional control of a promoter active in said plant. Currently, the more widely used DNA constructs are those that encode hairpin RNA (hpRNA). In these constructs, the fragment of the target gene is inversely repeated, with generally a spacer region between the repeats.

The inventors further disclose chimeric DNA constructs for silencing a *NADH-GoGAT* gene.

Such a chimeric DNA construct of comprises:

- a promoter functional in a plant cell;
- a DNA sequence of 200 to 1000 bp, preferably of 300 to 900 bp, consisting of a fragment of a cDNA encoding a NADH-GoGAT protein or of its complementary, or having at least 95% identity, and by order of increasing preference, at least 96%, 97%, 98% or 99 % identity with said fragment, said DNA sequence being placed under transcriptional control of said promoter.

According to a preferred embodiment, said chimeric DNA construct comprises:

- a first DNA sequence of 200 to 1000 bp, preferably of 300 to 900 bp, consisting of a fragment of a cDNA encoding a NADH-GoGAT protein, or having at least 95% identity, and by order of increasing preference, at least 96%, 97%, 98% or 99 % identity with said fragment;
- a second DNA sequence that is the complementary of said first DNA, said first and second sequences being in opposite orientations ;
- a spacer sequence separating said first and second sequence, such that these first and second DNA sequences are capable, when transcribed, of forming a single double-stranded RNA molecule.

The spacer can be a random fragment of DNA. However, preferably, one will use an intron which is spliceable by the target plant cell. Its size is generally 400 to 2000 nucleotides in length.

A large choice of promoters suitable for expression of heterologous genes in plants is available in the art. They can be chosen among those disclosed above.

DNA constructs for silencing a *NADH-GoGAT* gene as defined above generally also include a transcriptional terminator (for instance the 35S transcriptional terminator, or the nopaline synthase (Nos) transcriptional terminator).

These DNA constructs for silencing a *NADH-GoGAT* gene as defined above can be obtained and introduced in a host cell or organism by the well-known techniques of recombinant DNA and genetic engineering, such as those described above.

The inventors futher disclose wheat plant cells or wheat plants genetically modified by a DNA construct for silencing a *NADH-GoGAT* gene as defined above. The polynucleotide may be transiently expressed; it can also be incorporated in a stable extrachromosomal replicon, or integrated in the chromosome.

In particular the inventors disclose a transgenic wheat plant containing a transgene comprising a DNA construct for silencing a *NADH-GoGAT* gene as defined above.

Foregoing and other objects and advantages of the invention will become more apparent from the following detailed description and accompanying drawing. It is to be understood however that this foregoing detailed description is exemplary only and is not restrictive of the invention.

Figure 1 represents the linear regression observed between the *GoGAT* gene expression (expressed as $\Delta\Delta$CT) and the NNI status of the *Arche* (square) and *Soissons* (round) wheat genotypes for 29 leaf samples collected after flowering (respectively at Z75 and Z65).

Figure 2 shows the cloning strategy for pSC4Act-synGOGAT TaMod-SCV.

Figure 3 shows the cloning strategy for pAct-TaGOGAT-RNAi-66-SCV.

## EXAMPLE 1: EXPERIMENTAL VALIDATION OF THE *NADH-GoGAT* GENE IN NITROGEN USE EFFICIENCY (NUE) IN WHEAT

### 1) Materials & Methods

Wheat leaf samples were collected on 2 trials (La Minière and Boigneville stations - Arvalis Institut du Végétal; France): one in field for cultivar *Arche* and in green house for cultivar *Soissons.* Different nitrogen treatments were applied to lead to samples with a range of Nitrogen Nutrional Index (NNI) from 0.49 to 1.34 after flowering. During wheat culture, sampling has been done at 2 stages corresponding to the Zadoks scale: Z65 (Soissons) and Z75 (Arche).

Total RNAs were extracted from all the samples with the SV96 Total RNA Isolation System (Promega) according to the manufacturer instructions. RNA integrity was verified on the Agilent Bioanalyzer and presence of potential genomic DNA was checked by QPCR on RNA. In the absence of genomic DNA no amplification is expected from RNA.

For each sample 2 μg of total RNA were submitted to the reverse transcription using the High capacity reverse transcription kit (Applied Biosystems) and random primers in 100μl. RT reaction was then 1/10$^{th}$ diluted and 2μl of cDNA used for the amplification. Each RNA sample was submitted to 2 independent RT reactions for technical reproducibility evaluation.

Quantitative PCR was performed on a AB17900 machine (Applied Biosystems), using Applied Biosystems reagents. The PCR reactions consisted of a hot-start Taq Polymerase activation step of 95°C for 5 minutes, followed by 2 steps amplification cycles (denaturation 95°, 30sec, annealing/elongation 60°, 1min). Expression levels of mRNA for *NADH-GoGAT* gene were calculated using the Ct estimated by the SDS software (Applied Biosystems) and normalized across samples using 4 control genes. Relative expression was then considered as the ΔΔCt between *NADH-GoGAT* gene and the average of controls.

### 2) Results

In order to validate the role of the *NADH-GoGAT* gene in NUE, an experiment on two bread wheat genotypes, *i.e. Arche* and *Soissons,* was conducted. Twenty nine leaf samples for *Arche* and nine for *Soissons* were collected after flowering (respectively at Z75 and Z65). The N nutrition index (NNI) value was calculated (ranking from 0.49 to 1.34) for each sample. Moreover, for the same samples, RNA was extracted and the expression pattern of *GoGAT was* analysed through *qPCR* (ranking from 0 to 14 ΔΔCT) using sequence specific primers (forward: AATTCTGGAAGGAAGGGCTTG ; SEQ ID NO: 19; reverse: TTTGTATCCCTCGCGTATAGCTT ; SEQ ID NO: 20). The results are shown in Table 2 below.

Table 2: Level of expression (ΔΔCT) for *NADH-GoGat* gene on the chromosome 3B locus, and NNI for 29 leaf samples on *Arche* and *Soissons* genotypes.

| *Arche* | | |
|---|---|---|
| Sample name | NNI value | ΔΔCT value |
| Z75N1F1 | 0,49 | 8,99 |
| Z75N1F2 | 0,49 | 9,37 |
| Z75N2F1 | 0,66 | 7,91 |
| Z75N2F2 | 0,66 | 8,32 |
| Z75N2F3 | 0,66 | 7,52 |
| Z75N3F1 | 0,67 | 7,82 |
| Z75N3F2 | 0,67 | 8,14 |
| Z75N4F1 | 0,83 | 7,93 |
| Z75N4F2 | 0,83 | 7,51 |
| Z75N4F3 | 0,83 | 6,92 |
| Z75N5F1 | 0,74 | 7,62 |
| Z75N5F2 | 0,74 | 6,90 |
| Z75N5F3 | 0,74 | 7,10 |
| Z75N6F1 | 0,96 | 7,11 |

(continued)

| *Arche* | | |
|---|---|---|
| Sample name | NNI value | ΔΔCT value |
| Z75N6F2 | 0,96 | 6,71 |
| Z75N6F3 | 0,96 | 7,80 |
| Z75N7F1 | 1,12 | 6,73 |
| Z75N7F2 | 1,12 | 6,11 |
| Z75N8F1 | 1,25 | 6,99 |
| Z75N8F2 | 1,25 | 5,68 |
| | | |
| *Soissons* | | |
| Sample name | NNI score | ΔΔCT value |
| Z65_F1_T1 | 0,62 | 8,1 |
| Z65_F1_T2 | 0,99 | 7,5 |
| Z65_F1_T3 | 1,34 | 6,7 |
| Z65_F2_T1 | 0,62 | 6,7 |
| Z65_F2_T2 | 0,99 | 6,6 |
| Z65_F2_T3 | 1,34 | 6,4 |
| Z65_F3_T1 | 0,62 | 7,6 |
| Z65_F3_T2 | 0,99 | 6,7 |
| Z65_F3_T3 | 1,34 | 6,9 |

A significant correlation of $R^2$=63% and 37% was found between the expression (ΔΔCT values) of the *NADH-GoGAT* gene and the NNI score of the 29 leaves samples for both the *Arche* and *Soissons* genotypes, respectively. These results confirm that the *NADH-GoGAT* gene is the major candidate gene driving NUE on chromosome 3B (Figure 1).

**EXAMPLE 2: CONSTRUCTION OF TRANSGENIC WHEAT PLANTS OVEREXPRESSING A WHEAT NADH-GoGAT**

**1) <u>Wheat transformation constructs for NADH-GoGAT overexpression</u>**

The *Nco*I-*Xba*I synthetic fragment of the wheat *NADH-GOGAT* is cloned in the pUC57 vector (GenBank accession number: Y14837 (GI:2440162)), leading to the pUC57_synGOGAT TaMod vector. The *Nco*I-*Xba*I GOGAT fragment from pUC57_synGOGAT TaMod is then introduced in the pENTR4 vector (Invitrogen) linearised with *Nco*I-*Xba*I, to create the pENTR4_synGOGAT TaMod.

An LR clonase reaction between the pENTR4_synGOGAT TaMod and the pSC4Act-R1R2-SCV allows the creation of pSC4Act-synGOGAT TaMod-SCV (Figure 2). pSC4Act-R1R2-SCV is a vector using the Gateway approach to introduce genes to be expressed under the control of the rice *Actin* gene promoter (McElroy *et al.,* 1990). pSC4Act-R1R2-SCV is obtained after introduction of the proActin-R1 R2-terNOS cassette into the binary vector pSCV1 (Firek *et al.,* 1993). The binary plasmid pSC4Act-synGOGAT TaMod-SCV, is then introduced in the *A. tumefaciens* hypervirulent strain EHA 105, and used for transformation experiments.

**2) <u>Wheat transformation protocol</u>**

The method is essentially similar to the one described in International Application WO 00/63398. Wheat tillers, approximately 14 days post-anthesis (embryos approximately 1mm in length), are harvested from glasshouse grown plants to include 50cm tiller stem (22/15°C day/night temperature, with supplemented light to give a 16 hour day). All leaves are then removed except the flag leaf and the flag leaf is cleaned to remove contaminating fungal spores. The glumes of each spikelet and the lemma from the first two florets are then carefully removed to expose the immature seeds. Only these two seeds in each spikelet are generally uncovered. This procedure is carried out along the entire length of the inflorescence. The ears are then sprayed with 70% IMS as a brief surface sterilization.

*Agrobacterium tumefaciens* strains containing the vector for transformation are grown on solidified YEP media with 20mg/l kanamycin sulphate at 27°C for 2 days. Bacteria are then collected and re-suspended in TSIM1 (MS media with 100mg/l myoinositol, 10g/l glucose, 50mg/l MES buffer pH5.5) containing 400μM acetosyringone to an optical density of 2.4 at 650nm.

Agrobacterium suspension (1μl) is inoculated into the immature seed approximately at the position of the scutellum: endosperm interface, using a 10μl Hamilton, so that all exposed seed are inoculated. Tillers are then placed in water, covered with a translucent plastic bag to prevent seed dehydration, and placed in a lit incubator for 3 days at 23°C, 16hr day, 45 μEm-2s-1 PAR.

**[0086]** After 3 days of co-cultivation, inoculated immature seeds are removed and surface sterilized (30 seconds in 70% ethanol, then 20 minutes in 20% Domestos, followed by thorough washing in sterile distilled water). Immature embryos are aseptically isolated and placed on W4 medium (MS with 20g/l sucrose, 2mg/l 2,4-D, 500mg/l Glutamine, 100mg/l Casein hydrolysate, 150mg/l Timentin, pH5.8, solidified with 6g/l agarose) and with the scutellum uppermost. Cultures are placed at 25°C in the light (16 hour day). After 12 days cultivation on W4, embryogenic calli are transferred to W425G media (W4 with 25mg/l Geneticin (G418)). Calli are maintained on this media for 2 weeks and then each callus is divided into 2mm pieces and re-plated onto W425G.

After a further 2 week culture, all tissues are assessed for development of embryogenic callus: any callus showing signs of continued development after 4 weeks on selection is transferred to regeneration media MRM 2K 25G (MS with 20g/l sucrose, 2mg/l Kinetin, 25mg/l Geneticin (G418), pH5.8, solidified with 6g/l agarose). Shoots are regenerated within 4 weeks on this media and then transferred to MS20 (MS with 20g/l sucrose, pH5.8, solidified with 7g/l agar) for shoot elongation and rooting.

The presence of the T-DNA, and the number of copies are quantified by Quantitative PCR (qPCR).

## EXAMPLE 3: ASSOCIATION STUDIES

The aim of association studies is to identify loci contributing to quantitative traits, based on statistical association between genotypes and phenotypes using a large germplasm collection (panel) without knowledge on pedigree. At the opposite of linkage mapping, association studies can be performed using a selection of cultivars without the need for crossing and screening offspring. In this way, it can be looked at a maximum of genotypic variability (depending on panel selection) in a single study. Thus, using this technique, it is possible to identify favorable alleles of the *NADH-GoGAT* gene linked to phenotypic data, with a high resolution.

Two approaches can be used:

- whole genome scanning by searching polymorphism between markers spanning whole genome and phenotypic data without *a priori* on a genomic area of interest;
- after identification of QTL's *NADH-GoGAT* gene, a SNPs discovery is done in this gene, that are then linked to phenotypic data (the same traits of ones used for QTLs detection). Results expected are positive association between SNPs and phenotypic data to conclude on the implication of the gene in the QTL's effect (Linkage Disequilibrium in the area has to be considered).

Association study can provide information on gene polymorphisms implicated in traits and can indicate which allele is favorable regarding these traits.

## EXAMPLE 4: WHEAT RNAi TRANSFORMATION CONSTRUCTS FOR NADH-GoGAT REPRESSION

A 500 bp *Xba*I-*Xmn*I synthetic fragment (represented as SEQ ID NO: 21) of *NADH-GoGAT* is cloned in the pUC57 vector, leading to the pUC57_TaGOGAT vector. The *Xba*I-*Xmn*I GOGAT RNAi fragment from pUC57-TaGOGAT is then introduced in the pENTR1A vector (Invitrogen) linearised with *Xba*I-*Xmn*I, to create the pENTR1A_TaGOGAT.

An LR clonase reaction between the pENTR1A_TaGOGAT and the pAct-IR-66-SCV, allows the creation of pAct-TaGOGAT-RNAi-66-SCV (Figure 3). pAct-IR-66-SCV is a vector used to create RNAi vectors under the control of the rice *Actin* gene promoter (McElroy *et al.,* 1990). pAct-IR-66-SCV is obtained after introduction of the proActin-RNAi-terSac66 cassette from pBIOS890 into the binary vector pSCV 1 (Firek *et al.,* 1993). The binary plasmid pAct-TaGOGAT-RNAi-66-SCV is then introduced in the *A. tumefaciens* hypervirulent strain EHA 105, and used for transformation experiments.

## REFERENCES

- Agrama et al., (1999) Molecular Breeding 5:187-195.
- An et al., (1986) Plant Physiology 81:86-91.
- An et al., (2006) Plant and Soil 284:73-84.
- Bechtold et al., (1993), C R Acad Sci III 316:1194-1199.
- Bernard & Habash, (2009) New Phytologist 182:608-620.
- Bertin & Gallais (2001) Maydica 46:53-68.
- Borrell et al., (1998) Australian Journal of Agricultural Research 49:829-843.
- Cai et al., (2009) Plant Cell 28:527-537.
- Caputo et al., (2009) Plant Physiol. Biochem. 47:335-342.
- Chupeau et al., (1989) Biotechnology 7:503-508.
- Depicker et al., (1982J) Mol. Appl. Genet. 1:561-73.
- Finer et al., (1992) Plant Cell Report 11:323-328.
- Firek et al., (1993) Plant Mol Biol. 22:129-142.
- Fontaine et al., (2009) Theoretical and Applied Genetics 119:645-662.
- Fromm et al., (1990) Nature 319:791-793.
- Guerche et al., (1987) Biochimie 69:621-628.
- Goldsbrough et al., (1993) Biotechnology 11:1286-1292.
- Habash et al. (2007) Theoretical and Applied Genetics 114:403-419.
- Hayakawa et al., (1994) Planta 193:455-460.
- Hirel et al., (2001) Plant Physiology 125:1258-1270.
- Ireland & Lea eds, (1999) The enzymes of glutamine, glutamate,asparagine and aspartate metabolism (Marcel Dekker, New York), pp 49-109.
- Ishida et al., (1996) Nature Biotechnology 14:745-750.
- Ishiyama et al., (2004) Journal of Biological Chemistry 279:16598-16605.
- Jeanneau et al., (2002) Biochimie 84:1127-1135.
- Kasuga et al., (1999) Nature Biotech. 17:287-291.
- Katayama et al., (2000) Plant Mol. Biol. 44:99-106.
- Kjaer et al., (1995) Genome 38:1098-1104.
- Krapp et al., (2005) Photosynthesis Research 83:251-263.
- Laperche et al., (2006) Theoretical and Applied Genetics 112:797-807.
- Laperche et al., (2007) Theoretical and Applied Genetics 115:399-415.
- Le Gouis et al., (2000) European Journal of Agronomy 12:163-173.
- Lea & Miin, (2003) Plant Physiology and Biochemistry 41:555-564.
- Lian et al., (2005) Theoretical and Applied Genetics 112:85-96.
- Lima et al., (2006) Journal of Experimental Botany 57:2751-2761.
- Loudet et al., Plant Physiology 131:1508-1508.
- McCallum et al., (2000) Plant Physiology 123:439-442.
- McElroy et al., (1990) Plant Cell 2:163-171.
- McElroy et al., (1991) Mol. Gen. Genet. 231:150-160.
- Miin & Habash, (2002) Journal of experimental botany 53:979-987.
- Narusaka et al., (2003) Plant J. 34:137-148.
- Noh and Amasino, (1999) Plant Mol. Biol. 41:181-194.
- Obara, et al., (2001) Journal of Experimental Botany 52:1209-1217.
- Ochs et al., (1999) Plant Molecular Biology 39:395-405.
- Oury et al., (2003) Journal of Genetics & Breeding 57:59-68.
- Potel et al., (2009) Febs Journal 276:4061-4076.
- Rauh et al., (2002) Theoretical and Applied Genetics 104:743-750.
- Simmonds (1995) Journal of the Science of Food and Agriculture 67:309-315.
- Tabuchi et al., (2007) Journal of Experimental Botany 58:2319-2327.
- Thomas & Flavell, (1990) Plant Cell 2:1171-80.
- Valadier et al., (2008) Febs Journal 275:3193-3206.
- Verdaguer et al., (1996) Plant Mol. Biol. 6:1129-39.
- Watson et al., (2005) FEBS Letters 579: 5982-5987.
- Yamaya et al., (1992) Plant Physiology 100:1427-1432.

- Yamaya et al., (2002) Journal of Experimental Botany 53:917-925.

## Claims

1. A method for improving the grain filling of a wheat plant, wherein said method comprises overexpressing in said plant a NADH-dependent glutamate synthase (NADH-GoGAT) having at least 95% identity with the polypeptide of sequence SEQ ID NO: 1.

2. The method of claim 1, wherein the grain filling is improved by increasing the grain weight and/or the grain protein content.

3. The method of claim 1 or claim 2, wherein said NADH-GoGAT is encoded by a nucleotide sequence selected from the group consisting of SEQ ID NO: 2, SEQ ID NO: 3, SEQ ID NO: 4 and SEQ ID NO: 5.

4. A recombinant expression cassette comprising a polynucleotide encoding a NADH-GoGAT as defined in any of claims 1 or 3, under control of a heterologous promoter functional in a plant cell.

5. A recombinant vector containing an expression cassette comprising a polynucleotide encoding a NADH-GoGAT as defined in any of claims 1 or 3, under control of a promoter.

6. A host cell containing a recombinant expression cassette of claim 4 or a recombinant vector of claim 5.

7. A host cell of claim 6 which is a wheat plant cell.

8. A method for producing a transgenic wheat plant having an improved grain filling, wherein said method comprises:

   - providing a wheat plant cell of claim 7;
   - regenerating from said plant cell a transgenic wheat plant overexpressing a NADH-GoGAT as defined in any of claims 1 or 3.

9. A transgenic wheat plant obtainable by the method of claim 8.

**10.** A transgenic wheat plant or an isolated organ or tissue thereof comprising, stably integrated in its genome, a recombinant expression cassette comprising a polynucleotide encoding a NADH-GoGAT as defined in any of claims 1 or 3.

**11.** Wheat seeds containing a recombinant expression cassette of claim 4 obtained from a transgenic wheat plant of any of claims 9 or 10.

**12.** A wheat NADH-dependent glutamate synthase protein having at least 95% identity with the polypeptide of sequence SEQ ID NO: 1.

**13.** An isolated polynucleotide chosen from the group consisting of:

a) a polynucleotide encoding a wheat NADH-GoGAT, which polypeptide has at least 95% identity with the polypeptide of sequence SEQ ID NO: 1;
b) a polynucleotide complementary to the polynucleotide a);
c) a polynucleotide capable of hybridizing selectively, under stringent conditions, with the polynucleotide a) or the polynucleotide b).

**14.** A nucleic acid molecule according to claim 13, selected from the group consisting of SEQ ID NO: 2, SEQ ID NO: 3, SEQ ID NO: 4 and SEQ ID NO: 5.

**15.** A pair of primers, **characterized in that** it is selected from the group consisting of the sequences SEQ ID NO: 7 and SEQ ID NO: 8, SEQ ID NO: 9 and SEQ ID NO: 10, SEQ ID NO: 11 and SEQ ID NO: 12, SEQ ID NO: 13 and SEQ ID NO: 14, SEQ ID NO: 15 and SEQ ID NO: 16, SEQ ID NO: 17 and SEQ ID NO: 18, and SEQ ID NO: 19 and SEQ ID NO: 20.

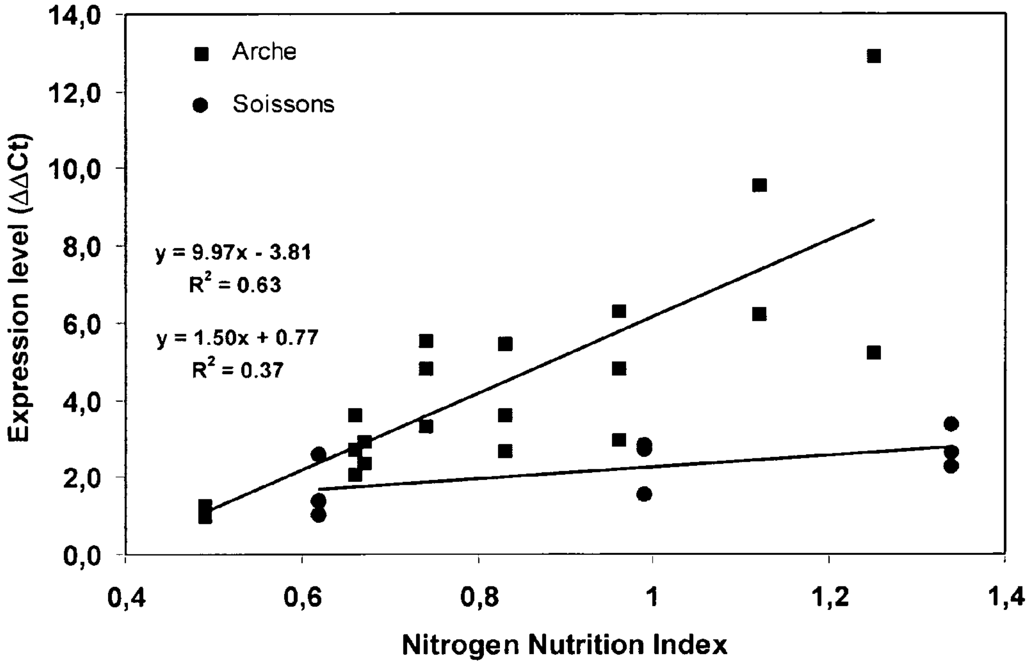
14,0
12,0
10,0
8,0
6,0
4,0
2,0
0,0
Expression level (ΔΔCt)
Arche
Soissons
y = 9.97x - 3.81
R² = 0.63
y = 1.50x + 0.77
R² = 0.37
0,4
0,6
0,8
1
1,2
1,4
Nitrogen Nutrition Index

**Figure 1**

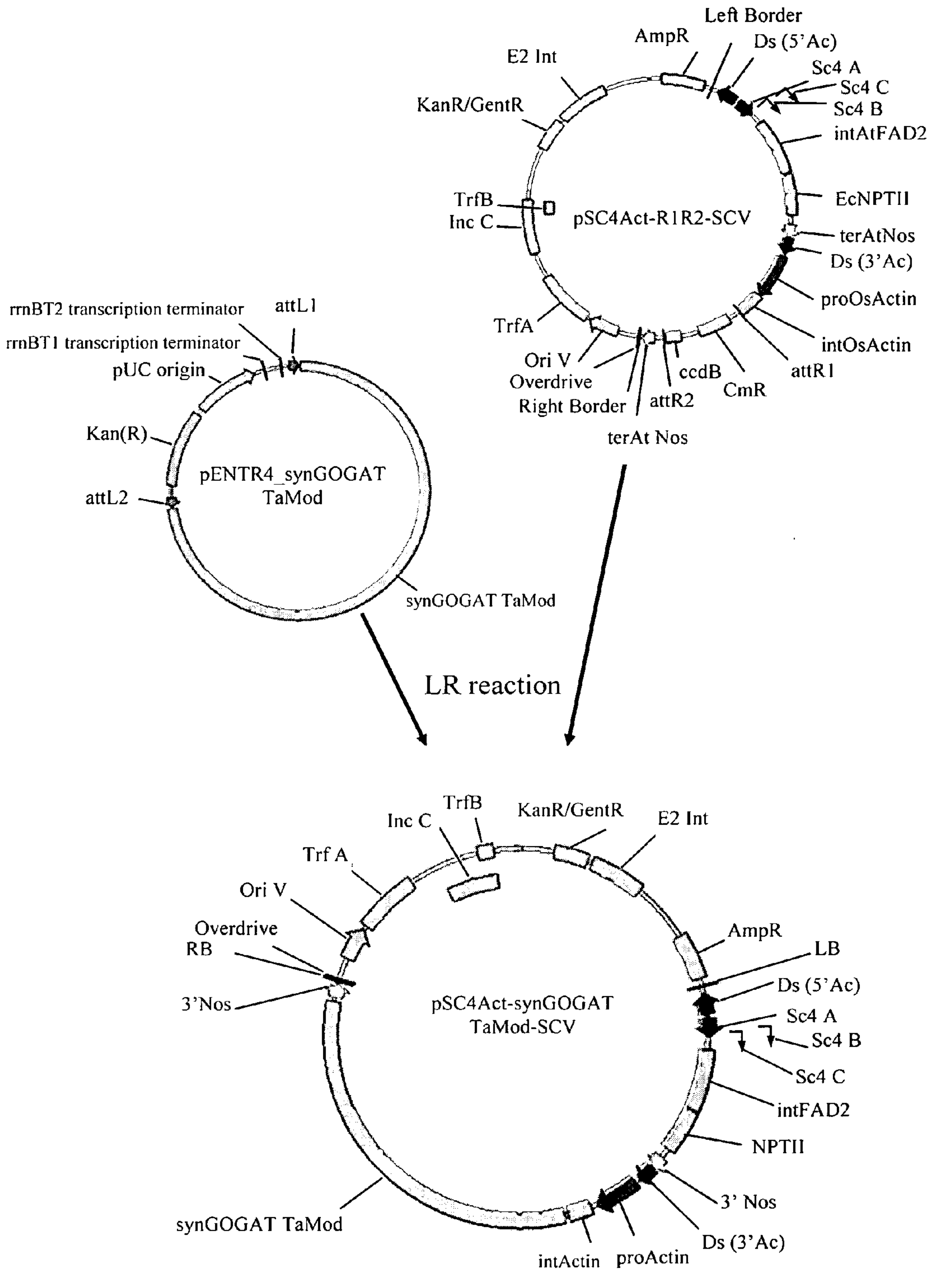
Left Border
AmpR
Ds (5'Ac)
E2 Int
Sc4 A
Sc4 C
Sc4 B
KanR/GentR
intAtFAD2
TrfB
Inc C
pSC4Act-R1R2-SCV
EcNPTII
terAtNos
Ds (3'Ac)
proOsActin
TrfA
intOsActin
Ori V
Overdrive
Right Border
ccdB
attR2
CmR
attR1
terAt Nos
rrnBT2 transcription terminator
attL1
rrnBT1 transcription terminator
pUC origin
Kan(R)
pENTR4_synGOGAT TaMod
attL2
synGOGAT TaMod
LR reaction
TrfB
Inc C
KanR/GentR
E2 Int
Trf A
Ori V
Overdrive
RB
AmpR
LB
Ds (5'Ac)
3'Nos
pSC4Act-synGOGAT TaMod-SCV
Sc4 A
Sc4 B
Sc4 C
intFAD2
NPTII
3' Nos
synGOGAT TaMod
Ds (3'Ac)
intActin
proActin

**Figure 2**

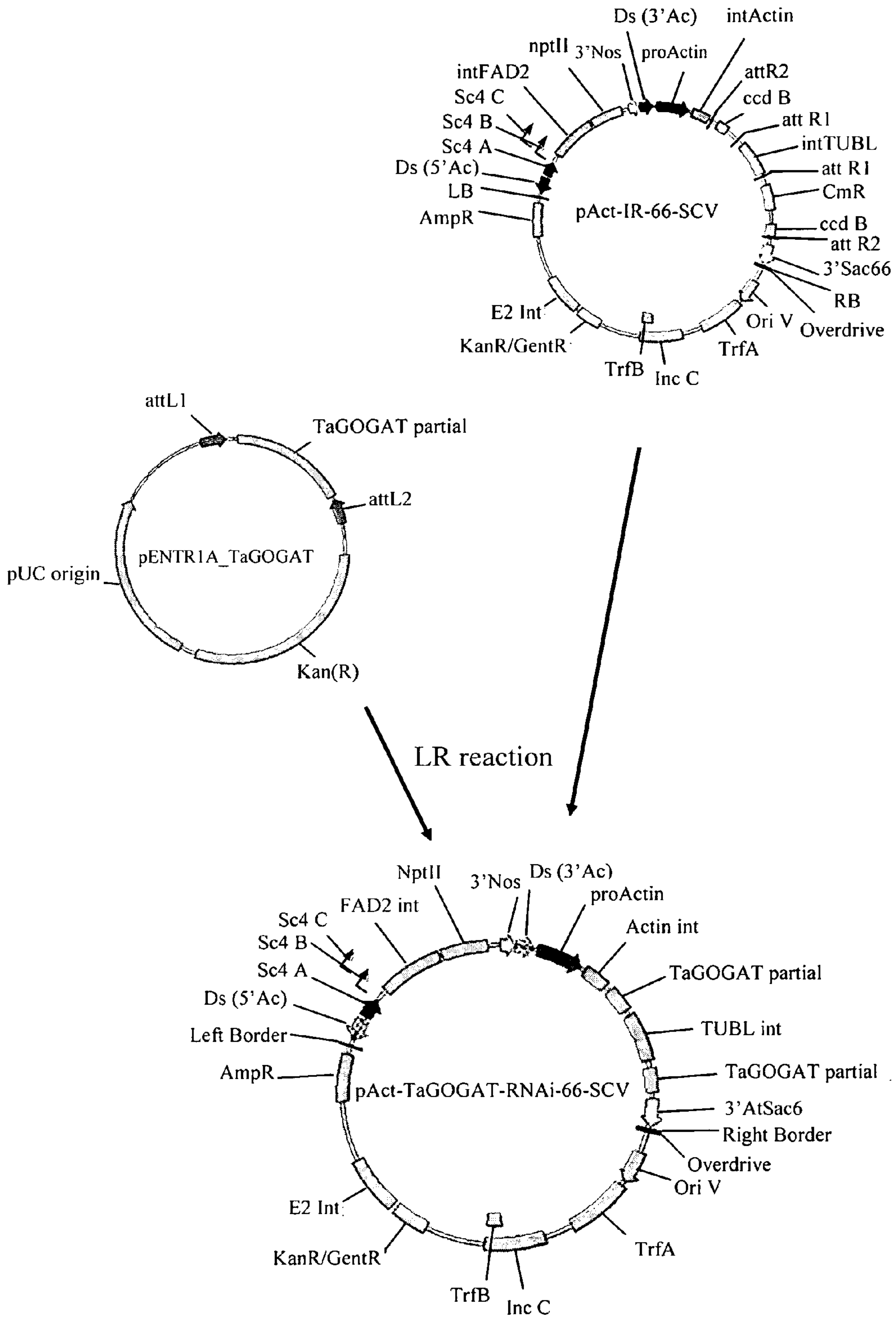
Ds (3'Ac)
intActin
nptII
3'Nos
proActin
intFAD2
attR2
Sc4 C
ccd B
Sc4 B
att R1
Sc4 A
intTUBL
Ds (5'Ac)
att R1
LB
CmR
AmpR
pAct-IR-66-SCV
ccd B
att R2
3'Sac66
RB
E2 Int
Ori V
Overdrive
KanR/GentR
TrfA
TrfB
Inc C
attL1
TaGOGAT partial
attL2
pENTR1A_TaGOGAT
pUC origin
Kan(R)
LR reaction
NptII
3'Nos
Ds (3'Ac)
proActin
FAD2 int
Sc4 C
Actin int
Sc4 B
Sc4 A
TaGOGAT partial
Ds (5'Ac)
TUBL int
Left Border
AmpR
TaGOGAT partial
pAct-TaGOGAT-RNAi-66-SCV
3'AtSac6
Right Border
Overdrive
Ori V
E2 Int
TrfA
KanR/GentR
TrfB
Inc C

**Figure 3**

Europäisches Patentamt
European Patent Office
Office européen des brevets

# EUROPEAN SEARCH REPORT

Application Number
EP 10 29 0058

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2004/123343 A1 (LA ROSA THOMAS J [US] ET AL) 24 June 2004 (2004-06-24)<br>* see SEQ ID NO: 15798 and 118281; paragraph [0020]; claims 1-3 *<br>* paragraph [0048] *<br>* paragraph [0114] *<br>----- | 1-15 | INV.<br>C12N15/82<br>C12N9/06 |
| X | YAMAYA TOMOYUKI ET AL: "Genetic manipulation and quantitative-trait loci mapping for nitrogen recycling in rice"<br>JOURNAL OF EXPERIMENTAL BOTANY,<br>vol. 53, no. 370, April 2002 (2002-04), pages 917-925, XP002585968<br>ISSN: 0022-0957<br>* the whole document *<br>----- | 1-3 | |
| X | BOISSON M ET AL: "Partial sequences of nitrogen metabolism genes in hexaploid wheat"<br>THEORETICAL AND APPLIED GENETICS ; INTERNATIONAL JOURNAL OF PLANT BREEDING RESEARCH, SPRINGER, BERLIN, DE LNKD- DOI:10.1007/S00122-004-1913-4,<br>vol. 110, no. 5, 1 March 2005 (2005-03-01), pages 932-940, XP019321868<br>ISSN: 1432-2242<br>* the whole document *<br>-----<br>-/-- | 13-15 | TECHNICAL FIELDS SEARCHED (IPC)<br>C12N |

The present search report has been drawn up for all claims

2

| Place of search | Date of completion of the search | Examiner |
|---|---|---|
| The Hague | 11 June 2010 | Oderwald, Harald |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 10 29 0058

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE EMBL [Online]<br>18 June 2009 (2009-06-18), "WRIS_4912 cDNA library of a compatible interaction between stripe rust (Puccinia striiformis) and wheat Triticum aestivum cDNA 5' similar to Os01g0681900, mRNA sequence."<br>XP002585794<br>retrieved from EBI accession no. EMBL:GR304543<br>Database accession no. GR304543<br>* abstract *<br>----- | 13 | |
| X | DATABASE EMBL [Online]<br>29 March 1997 (1997-03-29), "Oryza sativa gene for NADH-dependent glutamate synthase."<br>XP002585795<br>retrieved from EBI accession no. EMBL:AB001916<br>Database accession no. AB001916<br>* abstract * | 13 | |
| A | -& GOTO SATOSHI ET AL: "Organization and structure of NADH-dependent glutamate synthase gene from rice plants"<br>BIOCHIMICA ET BIOPHYSICA ACTA,<br>vol. 1387, no. 1-2,<br>8 September 1998 (1998-09-08), pages 298-308, XP002585969<br>ISSN: 0006-3002<br>* abstract *<br>-----<br>-/-- | 13 | TECHNICAL FIELDS SEARCHED (IPC) |

The present search report has been drawn up for all claims

| Place of search | Date of completion of the search | Examiner |
|---|---|---|
| The Hague | 11 June 2010 | Oderwald, Harald |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

2

EPO FORM 1503 03.82 (P04C01)

Europäisches Patentamt
European Patent Office
Office européen des brevets

# EUROPEAN SEARCH REPORT

Application Number
EP 10 29 0058

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GOOD A G ET AL: "Can less yield more? Is reducing nutrient input into the environment compatible with maintaining crop production?" TRENDS IN PLANT SCIENCE, ELSEVIER SCIENCE, OXFORD, GB LNKD- DOI:10.1016/J.TPLANTS.2004.10.008, vol. 9, no. 12, 1 December 2004 (2004-12-01), pages 597-605, XP004750080 ISSN: 1360-1385 * the whole document * ----- | 1 | |
| A | CHICHKOVA S ET AL: "Transgenic tobacco plants that overexpress alfalfa NADH-glutamate synthase have higher carbon and nitrogen content" JOURNAL OF EXPERIMENTAL BOTANY, OXFORD UNIVERSITY PRESS, GB, vol. 52, no. 364, 1 November 2001 (2001-11-01), pages 2079-2087, XP002387035 ISSN: 0022-0957 * the whole document * ----- | 1 | |

TECHNICAL FIELDS SEARCHED (IPC)

The present search report has been drawn up for all claims

| Place of search | Date of completion of the search | Examiner |
|---|---|---|
| The Hague | 11 June 2010 | Oderwald, Harald |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 10 29 0058

This annex lists the patent family members relating to the patent documents cited in the above-mentioned European search report.
The members are as contained in the European Patent Office EDP file on
The European Patent Office is in no way liable for these particulars which are merely given for the purpose of information.

11-06-2010

| Patent document cited in search report | Publication date | Patent family member(s) | Publication date |
|---|---|---|---|
| US 2004123343 A1 | 24-06-2004 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0063398 A
- US 5190065 A
- US 5283323 A


### Non-patent literature cited in the description


- **AGRAMA et al.** *Molecular Breeding,* 1999, vol. 5, 187-195
- **AN et al.** *Plant Physiology,* 1986, vol. 81, 86-91
- **AN et al.** *Plant and Soil,* 2006, vol. 284, 73-84
- **BECHTOLD et al.** *C R Acad Sci III,* 1993, vol. 316, 1194-1199
- **BERNARD ; HABASH.** *New Phytologist,* 2009, vol. 182, 608-620
- **BERTIN ; GALLAIS.** *Maydica,* 2001, vol. 46, 53-68
- **BORRELL et al.** *Australian Journal of Agricultural Research,* 1998, vol. 49, 829-843
- **CAI et al.** *Plant Cell,* 2009, vol. 28, 527-537
- **CAPUTO et al.** *Plant Physiol. Biochem.,* 2009, vol. 47, 335-342
- **CHUPEAU et al.** *Biotechnology,* 1989, vol. 7, 503-508
- **DEPICKER et al.** *Mol. Appl. Genet.,* 1982, vol. 1, 561-73
- **FINER et al.** *Plant Cell Report,* 1992, vol. 11, 323-328
- **FIREK et al.** *Plant Mol Biol.,* 1993, vol. 22, 129-142
- **FONTAINE et al.** *Theoretical and Applied Genetics,* 2009, vol. 119, 645-662
- **FROMM et al.** *Nature,* 1990, vol. 319, 791-793
- **GUERCHE et al.** *Biochimie,* 1987, vol. 69, 621-628
- **GOLDSBROUGH et al.** *Biotechnology,* 1993, vol. 11, 1286-1292
- **HABASH et al.** *Theoretical and Applied Genetics,* 2007, vol. 114, 403-419
- **HAYAKAWA et al.** *Planta,* 1994, vol. 193, 455-460
- **HIREL et al.** *Plant Physiology,* 2001, vol. 125, 1258-1270
- The enzymes of glutamine, glutamate,asparagine and aspartate metabolism. Marcel Dekker, 1999, 49-109
- **ISHIDA et al.** *Nature Biotechnology,* 1996, vol. 14, 745-750
- **ISHIYAMA et al.** *Journal of Biological Chemistry,* 2004, vol. 279, 16598-16605
- **JEANNEAU et al.** *Biochimie,* 2002, vol. 84, 1127-1135
- **KASUGA et al.** *Nature Biotech.,* 1999, vol. 17, 287-291
- **KATAYAMA et al.** *Plant Mol. Biol.,* 2000, vol. 44, 99-106
- **KJAER et al.** *Genome,* 1995, vol. 38, 1098-1104
- **KRAPP et al.** *Photosynthesis Research,* 2005, vol. 83, 251-263
- **LAPERCHE et al.** *Theoretical and Applied Genetics,* 2006, vol. 112, 797-807
- **LAPERCHE et al.** *Theoretical and Applied Genetics,* 2007, vol. 115, 399-415
- **LE GOUIS et al.** *European Journal of Agronomy,* 2000, vol. 12, 163-173
- **LEA ; MIIN.** *Plant Physiology and Biochemistry,* 2003, vol. 41, 555-564
- **LIAN et al.** *Theoretical and Applied Genetics,* 2005, vol. 112, 85-96
- **LIMA et al.** *Journal of Experimental Botany,* 2006, vol. 57, 2751-2761
- **LOUDET et al.** *Plant Physiology,* vol. 131, 1508-1508
- **MCCALLUM et al.** *Plant Physiology,* 2000, vol. 123, 439-442
- **MCELROY et al.** *Plant Cell,* 1990, vol. 2, 163-171
- **MCELROY et al.** *Mol. Gen. Genet.,* 1991, vol. 231, 150-160
- **MIIN ; HABASH.** *Journal of experimental botany,* 2002, vol. 53, 979-987
- **NARUSAKA et al.** *Plant J.,* 2003, vol. 34, 137-148
- **NOH ; AMASINO.** *Plant Mol. Biol.,* 1999, vol. 41, 181-194
- **OBARA et al.** *Journal of Experimental Botany,* 2001, vol. 52, 1209-1217
- **OCHS et al.** *Plant Molecular Biology,* 1999, vol. 39, 395-405

- **OURY et al.** *Journal of Genetics & Breeding,* 2003, vol. 57, 59-68
- **POTEL et al.** *Febs Journal,* 2009, vol. 276, 4061-4076
- **RAUH et al.** *Theoretical and Applied Genetics,* 2002, vol. 104, 743-750
- **SIMMONDS.** *Journal of the Science of Food and Agriculture,* 1995, vol. 67, 309-315
- **TABUCHI et al.** *Journal of Experimental Botany,* 2007, vol. 58, 2319-2327
- **THOMAS ; FLAVELL.** *Plant Cell,* 1990, vol. 2, 1171-80
- **VALADIER et al.** *Febs Journal,* 2008, vol. 275, 3193-3206
- **VERDAGUER et al.** *Plant Mol. Biol.,* 1996, vol. 6, 1129-39
- **WATSON et al.** *FEBS Letters,* 2005, vol. 579, 5982-5987
- **YAMAYA et al.** *Plant Physiology,* 1992, vol. 100, 1427-1432
- **YAMAYA et al.** *Journal of Experimental Botany,* 2002, vol. 53, 917-925

SEQUENCE LISTING

<110> GENOPLANTE-VALOR

<120> IMPROVEMENT OF THE GRAIN FILLING OF WHEAT THROUGH THE MODULATION OF NADH-GLUTAMATE SYNTHASE ACTIVITY

<130> MJP/XRN/11-F1516/37

<160> 21

<170> PatentIn version 3.3

<210> 1

<211> 2144

<212> PRT

<213> Triticum aestivum

<400> 1

```
Met Pro Thr Ala Gln Gly Asp Trp Phe Glu Ala Ala Ala Pro Pro Gly
1               5                   10                  15
Gly Arg Arg Ala Arg Arg Ser Gln Ser Ala Ser Ala Pro Cys Arg Ser
            20                  25                  30
Ala Arg Gln Ala His Gly Ala Met Ser Leu Asp Gly Gly Phe Leu Gly
        35                  40                  45
Gly Ala Gln Arg Thr Glu Glu Arg Val Ala Pro Arg Pro Pro Arg Ala
    50                  55                  60
Ala Ala Arg Asp Ala Glu Ser Ile Arg Pro Met Ser Leu Leu Pro Glu
65                  70                  75                  80
Ser Ser Ile Gly Leu Tyr Asp Pro Ala Phe Glu Arg Asp Ser Cys Gly
                85                  90                  95
Val Gly Phe Val Ala Glu Leu Ser Gly Val Asp Asn Arg Ala Thr Val
            100                 105                 110
Val Asp Ala Ile Gln Met Leu Glu Arg Met Ala His Arg Gly Ala Cys
        115                 120                 125
Gly Cys Glu Lys Asn Thr Gly Asp Gly Ala Gly Ile Leu Val Ala Leu
    130                 135                 140
Pro His Thr Phe Phe Arg Glu Val Thr Lys Asp Ala Gly Phe Glu Leu
145                 150                 155                 160
Pro Pro Pro Gly Glu Tyr Ala Val Gly Met Val Phe Leu Pro Thr Asp
                165                 170                 175
Glu Lys Arg Arg Glu Arg Ser Lys Thr Glu Phe Thr Lys Val Ala Glu
            180                 185                 190
```

```
Ser Leu Gly His Ser Ile Leu Gly Trp Arg Gln Val Pro Thr Asp Asn
        195                 200                 205
Ser Asp Leu Gly Gln Ala Ala Leu Asp Thr Glu Pro Ala Ile Glu Gln
    210                 215                 220
Val Phe Leu Thr Lys Ser Ser Lys Ser Lys Ala Asp Phe Glu Gln Gln
225                 230                 235                 240
Val Phe Ile Leu Arg Arg Leu Ser Ile Val Ser Ile Arg Ala Ala Leu
                245                 250                 255
Asn Leu Gln Arg Gly Gly Glu Arg Asp Phe Tyr Met Cys Ser Leu Ser
            260                 265                 270
Ser Arg Thr Ile Val Tyr Lys Gly Gln Leu Met Pro Ser Gln Leu Gln
        275                 280                 285
Gly Tyr Tyr Tyr Ala Asp Ile Gly His Glu Asn Phe Ser Ser Tyr Met
    290                 295                 300
Ala Leu Val His Ser Arg Phe Ser Thr Asn Thr Phe Pro Ser Trp Asp
305                 310                 315                 320
Arg Ala Gln Pro Met Arg Val Leu Gly His Asn Gly Glu Ile Asn Thr
                325                 330                 335
Leu Lys Gly Asn Lys Asn Trp Met Lys Ala Arg Glu Gly Leu Leu Glu
            340                 345                 350
Cys Glu Lys Leu Gly Leu Ser Gln Asp Glu Met Ser Lys Ile Leu Pro
        355                 360                 365
Ile Val Asp Ala Thr Ser Ser Asp Ser Gly Ala Phe Asp Gly Val Leu
    370                 375                 380
Glu Leu Leu Ile Arg Gly Gly Arg Ser Leu Pro Glu Ala Val Met Met
385                 390                 395                 400
Met Ile Pro Glu Ala Trp Gln Asn Asp Val Asn Met Glu Pro Asp Lys
                405                 410                 415
Lys Ala Leu Tyr Glu Phe Leu Ser Ala Leu Met Glu Pro Trp Asp Gly
            420                 425                 430
Pro Ala Leu Ile Ser Phe Thr Asp Gly Arg Tyr Leu Gly Ala Thr Leu
        435                 440                 445
Asp Arg Asn Gly Leu Arg Pro Gly Arg Phe Tyr Val Thr His Ser Gly
    450                 455                 460
Arg Val Val Met Gly Ser Glu Val Gly Val Val Asp Ile Pro Ala Gln
465                 470                 475                 480
Asp Val Leu Arg Lys Gly Arg Leu Asn Pro Gly Met Met Leu Leu Val
                485                 490                 495
```

Asp Phe Asp Asn His Thr Val Val Asp Asp Glu Ala Leu Lys Ala Gln
500 505 510
Tyr Ser Lys Ala His Pro Tyr Gly Glu Trp Leu Lys Arg Gln Lys Met
515 520 525
Tyr Leu Lys Asp Ile Val Glu Ser Val Pro Glu Thr Asp Arg Val Ala
530 535 540
Pro Ser Ile Ser Gly Ser Ile Thr Gln Thr Asn Glu Asn Lys Glu Cys
545 550 555 560
Val Gly Ile Asn Ala Ile Val Thr Pro Leu Lys Ala Phe Gly Tyr Thr
565 570 575
Leu Glu Ala Leu Glu Met Leu Leu Leu Pro Met Ala Lys Asp Gly Val
580 585 590
Glu Ala Leu Gly Ser Met Gly Asn Asp Ala Pro Leu Ala Val Met Ser
595 600 605
Asn Arg Glu Lys Leu Thr Phe Glu Tyr Phe Lys Gln Met Phe Ala Gln
610 615 620
Val Thr Asn Pro Pro Ile Asp Pro Ile Arg Glu Lys Ile Val Thr Ser
625 630 635 640
Met Glu Cys Met Ile Gly Pro Glu Gly Asp Leu Leu Glu Ile Thr Glu
645 650 655
Lys Gln Cys Asn Arg Leu Ala Leu Lys Gly Pro Leu Val Ser Met Asp
660 665 670
Glu Met Glu Ser Ile Lys Lys Met Asn Tyr Arg Gly Trp Arg Ser Lys
675 680 685
Val Leu Asp Ile Thr Tyr Pro Lys Asn Ser Gly Arg Lys Gly Leu Glu
690 695 700
Glu Thr Leu Asp Arg Ile Cys Ala Glu Ala Arg Glu Ala Ile Arg Glu
705 710 715 720
Gly Tyr Lys Ile Leu Val Leu Ser Asp Arg Gly Phe Ser Ser Asp Arg
725 730 735
Val Ala Val Ser Ser Leu Leu Ala Val Gly Ala Val His Gln His Leu
740 745 750
Val Ala Asn Leu Glu Arg Thr Arg Val Gly Leu Leu Val Glu Ser Ala
755 760 765
Glu Pro Arg Glu Val His His Phe Cys Thr Leu Val Gly Phe Gly Ala
770 775 780
Asp Ala Ile Cys Pro Tyr Leu Ala Ile Glu Ala Ile Trp Cys Leu Gln
785 790 795 800

Thr Asp Gly Lys Ile Pro Pro Thr Asp Ser Lys Glu Glu Leu Val Glu
805 810 815

Lys Tyr Phe Tyr Ala Ser Ile Tyr Gly Met Met Lys Val Leu Ala Lys
820 825 830

Met Gly Ile Ser Thr Leu Ala Ser Tyr Lys Gly Ala Gln Ile Phe Glu
835 840 845

Ala Leu Gly Leu Ser Ser Glu Val Ile His Lys Cys Phe Glu Gly Thr
850 855 860

Pro Ser Arg Ile Glu Gly Ala Thr Phe Glu Met Leu Ala Arg Asp Ala
865 870 875 880

Leu Arg Leu His Glu Leu Ala Phe Pro Ser Arg Thr Pro Pro Pro Gly
885 890 895

Ser Ala Asp Ala Lys Ala Leu Pro Asn Pro Gly Asp Tyr His Trp Arg
900 905 910

Lys Asn Gly Glu Val His Leu Asn Asp Pro Leu Ala Met Ala Lys Leu
915 920 925

Gln Glu Ala Ala Lys Val Asn Ser Arg Glu Ala Tyr Lys Glu Tyr Ser
930 935 940

Lys Arg Ile Gln Glu Leu Asn Lys Ala Cys Asn Leu Arg Gly Met Leu
945 950 955 960

Lys Phe Ile Asp Ser Thr Ser Lys Ile Ser Leu Asp Glu Val Glu Pro
965 970 975

Ala Ser Glu Ile Val Lys Arg Phe Cys Thr Gly Ala Met Ser Tyr Gly
980 985 990

Ser Ile Ser Leu Glu Ala His Thr Ala Leu Ala Val Ala Met Asn Lys
995 1000 1005

Leu Gly Gly Lys Ser Asn Thr Gly Glu Gly Gly Glu Gln Pro Ser
1010 1015 1020

Arg Met Glu Pro Leu Pro Asp Gly Ser Met Asn Pro Lys Arg Ser
1025 1030 1035

Ala Ile Lys Gln Val Ala Ser Gly Arg Phe Gly Val Ser Ser Tyr
1040 1045 1050

Tyr Leu Thr Asn Ala Asp Gly Leu Gln Ile Lys Met Ala Gln Gly
1055 1060 1065

Ala Lys Pro Gly Glu Gly Gly Glu Leu Pro Gly His Lys Val Ile
1070 1075 1080

Gly Asp Ile Ala Val Thr Arg His Ser Thr Ala Gly Val Gly Leu
1085 1090 1095

Ile Ser Pro Pro Pro His His Asp Ile Tyr Ser Ile Glu Asp Leu
1100 1105 1110

Ala Gln Leu Ile His Asp Leu Lys Asn Ser Asn Pro Gln Ala Arg
1115 1120 1125

Ile Ser Val Lys Leu Val Ser Glu Ala Gly Val Gly Val Val Ala
1130 1135 1140

Ser Gly Val Val Lys Gly His Ala Asp His Val Leu Ile Ser Gly
1145 1150 1155

His Asp Gly Gly Thr Gly Ala Ser Arg Trp Thr Gly Ile Lys Asn
1160 1165 1170

Ala Gly Leu Pro Trp Glu Leu Gly Leu Ala Glu Thr His Gln Thr
1175 1180 1185

Leu Val Ala Asn Gly Leu Arg Gly Arg Ala Val Leu Gln Thr Asp
1190 1195 1200

Gly Gln Leu Lys Thr Gly Arg Asp Val Ala Val Ala Cys Leu Leu
1205 1210 1215

Gly Ala Glu Glu Phe Gly Phe Ser Thr Ala Pro Leu Ile Thr Leu
1220 1225 1230

Gly Cys Ile Met Met Arg Lys Cys His Thr Asn Thr Cys Pro Val
1235 1240 1245

Gly Ile Ala Thr Gln Asp Pro Val Leu Arg Glu Lys Phe Ala Gly
1250 1255 1260

Glu Pro Glu His Val Ile Asn Phe Phe Phe Met Leu Ala Glu Glu
1265 1270 1275

Leu Arg Glu Ile Met Ala Gln Leu Gly Leu Arg Thr Ile Asn Glu
1280 1285 1290

Met Val Gly Arg Ser Asp Met Leu Glu Val Asp Pro Glu Val Val
1295 1300 1305

Lys Ser Asn Glu Lys Leu Glu Asn Ile Asp Leu Ser Leu Ile Leu
1310 1315 1320

Lys Pro Ala Ala Glu Ile Arg Pro Gly Ala Ala Gln Tyr Cys Val
1325 1330 1335

Glu Lys Gln Asp His Gly Leu Asp Met Ala Leu Asp Asn Lys Leu
1340 1345 1350

Ile Ala Leu Ser Arg Ala Ala Leu Glu Lys Glu Val Arg Val Phe
1355 1360 1365

Ile Glu Thr Pro Ile Lys Asn Thr Asn Arg Ala Val Gly Thr Thr
1370 1375 1380

Leu Ser His Glu Val Thr Lys Arg Tyr His Met Lys Gly Leu Asp
1385 1390 1395

Pro Gly Thr Ile His Val Lys Leu Thr Gly Ser Ala Gly Gln Ser
1400 1405 1410

Phe Gly Ala Phe Leu Cys Pro Gly Ile Thr Leu Glu Leu Glu Gly
1415 1420 1425

Asp Ser Asn Asp Tyr Val Gly Lys Gly Leu Ser Gly Gly Lys Ile
1430 1435 1440

Val Val Tyr Pro Pro Arg Asn Ser Thr Phe Ser Ala Glu Asp Asn
1445 1450 1455

Ile Val Ile Gly Asn Val Ala Leu Tyr Gly Ala Thr Lys Gly Glu
1460 1465 1470

Ala Tyr Phe Asn Gly Met Ala Ala Glu Arg Phe Cys Val Arg Asn
1475 1480 1485

Ser Gly Ala Arg Thr Val Val Glu Gly Ile Gly Asp His Gly Cys
1490 1495 1500

Glu Tyr Met Thr Gly Gly Thr Val Val Ile Leu Gly Lys Thr Gly
1505 1510 1515

Arg Asn Phe Ala Ala Gly Met Ser Gly Gly Ile Ala Tyr Val Tyr
1520 1525 1530

Asp Val Asp Gly Thr Phe Ser Val Arg Cys Asn Asn Glu Leu Val
1535 1540 1545

Asp Leu Tyr His Val Glu Glu Glu Asp Asp Val Thr Thr Leu Lys
1550 1555 1560

Met Met Ile Glu Gln His Arg Leu His Thr Glu Ser Val Leu Ala
1565 1570 1575

Lys Asp Ile Leu Ser Lys Phe Asp Thr Leu Leu Pro Lys Phe Val
1580 1585 1590

Lys Val Tyr Pro Arg Asp Tyr Lys Arg Val Leu Glu Glu Met Lys
1595 1600 1605

Ala Glu Lys Ala Ala Ala Arg Pro Thr Lys Glu Pro Lys Val Ala
1610 1615 1620

Asn Gly Val Ser Val Thr Thr Lys Lys Ile Gln Thr Glu Lys Ser
1625 1630 1635

Ser Ser Arg Pro Thr Arg Val Ala Asn Ala Lys Lys Tyr Arg Gly
1640 1645 1650

Phe Val Thr Tyr Glu Arg Glu Gly Val Ser Tyr Arg Asp Pro Asn
1655 1660 1665

Glu Arg Val Lys Asp Trp Asn Glu Val Ala Ile Glu Ser Val Pro
1670 1675 1680
Gly Pro Leu Leu Asn Thr Gln Ser Ala Arg Cys Met Asp Cys Gly
1685 1690 1695
Thr Pro Phe Cys His Gln Glu Ser Ser Gly Ala Gly Cys Pro Leu
1700 1705 1710
Gly Asn Lys Ile Pro Glu Phe Asn Glu Leu Val His Gln Asn Arg
1715 1720 1725
Trp Arg Glu Ala Leu Asp Arg Leu Leu Glu Thr Asn Asn Phe Pro
1730 1735 1740
Glu Phe Thr Gly Arg Val Cys Pro Ala Pro Cys Glu Gly Ser Cys
1745 1750 1755
Val Leu Gly Ile Ile Glu Asn Pro Val Ser Ile Lys Ser Ile Glu
1760 1765 1770
Cys Ser Ile Ile Asp Lys Gly Phe Glu Glu Gly Trp Met Val Pro
1775 1780 1785
Arg Pro Pro Leu Gln Arg Thr Gly Lys Lys Ile Ala Ile Val Gly
1790 1795 1800
Ser Gly Pro Ala Gly Leu Ala Ala Ala Asp Gln Leu Asn Lys Met
1805 1810 1815
Gly His Phe Val Thr Val Phe Glu Arg Ser Asp Arg Ile Gly Gly
1820 1825 1830
Leu Met Met Tyr Gly Val Pro Asn Met Lys Thr Asp Lys Ile Gly
1835 1840 1845
Val Val Gln Arg Arg Val Asn Leu Met Ala Glu Glu Gly Val Thr
1850 1855 1860
Phe Val Val Asn Ala Asn Val Gly Ser Asp Pro Leu Tyr Ser Ile
1865 1870 1875
Glu Arg Leu His Ser Glu Asn Asn Ala Val Ile Leu Ala Cys Gly
1880 1885 1890
Ala Thr Lys Pro Arg Asp Leu Ser Ile Pro Gly Arg Glu Leu Ala
1895 1900 1905
Gly Val His Phe Ala Met Glu Phe Leu His Ala Asn Thr Lys Ser
1910 1915 1920
Leu Leu Asp Ser Asn Leu Glu Asp Gly Arg Tyr Ile Ser Ala Gln
1925 1930 1935
Gly Lys Lys Val Val Val Ile Gly Gly Gly Asp Thr Gly Thr Asp
1940 1945 1950

Cys Ile Gly Thr Ser Val Arg His Gly Cys Ser Ser Ile Val Asn
1955 1960 1965

Leu Glu Leu Leu Thr Lys Pro Pro Ser Lys Arg Ala Ser Asp Asn
1970 1975 1980

Pro Trp Pro Gln Trp Pro Arg Val Phe Arg Val Asp Tyr Gly His
1985 1990 1995

Gln Glu Ala Ser Thr Lys Phe Gly Asn Asp Pro Arg Thr Tyr Glu
2000 2005 2010

Val Leu Thr Lys Arg Phe Ile Gly Asp Glu Asp Gly Lys Leu Lys
2015 2020 2025

Ala Leu Glu Val Val Arg Val Lys Trp Glu Lys Val Asp Gly Lys
2030 2035 2040

Phe Gln Phe Lys Glu Ile Glu Gly Ser Gln Glu Ile Ile Glu Ala
2045 2050 2055

Asp Leu Val Leu Leu Ala Met Gly Phe Leu Gly Pro Glu Glu Asn
2060 2065 2070

Ile Ala Asp Lys Leu Gly Leu Glu Lys Asp Asn Arg Ser Asn Phe
2075 2080 2085

Lys Ala Gln Phe Gly His Phe Gly Thr Ser Val Asp Gly Val Phe
2090 2095 2100

Ala Ala Gly Asp Cys Arg Arg Gly Gln Ser Leu Val Val Trp Ala
2105 2110 2115

Ile Thr Glu Gly Arg Glu Ala Ala Ala Ala Val Asp Lys Tyr Leu
2120 2125 2130

Ser Arg Asp Glu Gln Asn Val Ala Gly Leu Thr
2135 2140

<210> 2
<211> 10173
<212> DNA
<213> Triticum aestivum
<400> 2

atgccgacgg cgcaggggga ttggtttgaa gcacgcggcg ccgccgggcg gccgcagggc 60
ccgccgcagc cagtctgcct cggcgccgtg ccgctccgca aggcaggcgc acggcgccat 120
gtccctggat ggcgggttcc tcggcggcgc gcagcgcacc gaggaacgcg tcgcgccacg 180
cccgcctcgg gccgcggcgc gcgacgccga gtccatcagg cccatgtctc tgctacccga 240
gagcagcatt gggctgtacg acccggcgtt cgagcgtgac tcgtgcggcg ttggcttcgt 300

cgccgagctg tcgggcgttg acaaccgggc gaccgtgagt tctttgcacc aggacaccgc 360
cgcagcttct ctcttttatc taccacgttg agatattgat tttgctgtga tttactttac 420
aggtcgtcga tgccattcag atgcttgaaa gaatggcaca ccgaggtgcc tgcggctgtg 480
agaaaaacac tggtgatggt gccggcattc tcgttgctct accacacacc ttcttccgag 540
aggtgaataa acagaaaatt tcaagcaaac tcagttttcc ttgacagagt tattcatctt 600
gggtgttgct ttgctattgc tgcaggtgac aaaggatgcc ggtttcgagt taccgccacc 660
aggtgagtat gctgttggaa tggtcttcct gccaaccgat gagaagcgcc gcgagaggag 720
caaaactgag tttacaaagg ttggttggtt ggtgtataga caattagaca gcgtgcgagg 780
cgagtctgcg atggtaagct tatgtcttgg attgttttta ttcatgcagg tcgctgagtc 840
tctaggacat tcgatacttg ggtggcgcca ggttcccact gacaattcag acttgggcca 900
agctgcgctc gatactgaac cagccattga acaggttttc ctcaccaaga gttcaaaatc 960
gaaggccgac ttcgaacagc aggtcctcca tgagtttaat tttctctcaa cacatgtaat 1020
gagctttcct ctcaactaac ccatcaatta tcacagttgt ttatcctgag gaggctctca 1080
attgtatcta tccgggccgc gctgaatctt cagcgtggag gagagagaga tttctacatg 1140
tgctctctat cttcaaggtc cttacttctt ctgaattgca ttttcactac aaactgatga 1200
ggttgagatc agggagccca attaagctcc tgactttaat ggaacattaa caacacaaaa 1260
gttgatagga aagctgactg ggaaaaacaa tcctgggcta tctcttaagc attagaaatc 1320
tgtaatgtaa ggacatttca tttttgctgt attcatgtga gtaattattg ttaaatcttc 1380
tattgtggtt ttccaggacc attgtttaca agggccaact tatgccatcc cagctccaag 1440
ggtactacta tgcggatata ggtcatgaaa acttctccag ttatatggct ctggtaaata 1500
cgaaaccttg atactcttac aacaaaagtc atggtcgtag tcagcggatg ggaatcaata 1560
atagcaagcc agttgtaaga tcaagactcg ctcctgtgca gacatcaaat ttgagctgtc 1620
tattcatatg ctatttcagt cctgcttcgt gatgccttta agtgaagtac tagaaacttc 1680
tcttagttga tacccgataa ccatgttgca gagtccagct gcagttctgc tatggccgac 1740
tgcttgccga ctcttcagga tgtgaccgaa gaaattgtgt ttggtttgag gtagcttagg 1800
gctcccaact gttttgcatg aaattatacg cagctgacgt atttttacaa aattcgttct 1860
tcacaaggaa gttttgtttt gaatatctgc aaaagatgtt tataatatga tttctaaaag 1920
tcatatcttg aaacaggttc actcaaggtt ctccaccaac accttcccca gctgggaccg 1980
tgcacagcca atgcgtgtct taggccacaa tggagagatc aatactctca aagggaacaa 2040
aaactggtaa tattattgtc aaatcttttc ctctttgctt taacctttc ttggcagatg 2100
caactatctc aatatgcacg ttgtatatat ggcaggatga aagctcgtga gggtctcttg 2160
gagtgtgaga agcttggcct atcacaggat gaaatgtcaa agattcttcc aatagtagat 2220
gccacttctt cagattcagg tccgcagtct aatatgcagc atgaattcca cttggcaaat 2280
aaaaattcat ctcggttttg tgagttacta agtgtgaaaa aacatgtgaa ttctaggtgc 2340
atttgatggt gttctcgagc tccttatccg tggtggaaga agcctgccag aagctgtgat 2400
gatgatgatc cctgaggcgt ggcagaatga tgtaaacatg gaacctgata agaaagctct 2460
gtacgagttc ttgtcagccc ttatggagcc ttgggatgga cctgctctca tatcttgtaa 2520
aaaacttaaa cccgtttctt ttttctgatc atacttctaa ctgattactt aagctaacat 2580

gggtacaacc tgtagttacc gatggccgct accttggagc taccctggat cgcaatggcc 2640
ttaggcctgg tcgattttat gtaacccaca gtggacgtgt ggtcatgggt agtgaagttg 2700
gtgttgtgga tattcctgcc caagatgtgt tgagaaaggg tcggcttaac cctggaatga 2760
tgttactcgt tgacttcgac aaccatactg tagtagatga tgaagcactc aaggcacagt 2820
actctaaagc tcacccgtat ggagaatggc tcaagcgaca aaagatgtac ctcaaagaca 2880
ttgtagaatc tgtcccagaa actgacagag ttgctccaag catttctggt tctattacgg 2940
taagtactta cgtggtccaa cttcctcatt cactgggtag tttggtgatg aaaattggta 3000
aaccataagg aacatggtga ttttaatatt cttttacaat ttggtgtagc aaacaaatga 3060
gaacaaggaa tgtgtaggca tcaatgcaat tgtgactcca ctaaaggcgt ttgggtaagc 3120
aaggattgaa cattgtcaac tatcactcat gtttcaattt ctgctggcta atgatccaac 3180
cgattaacta caggtacaca ttggaagccc tagaaatgtt gctgctgcca atggctaaag 3240
atggagtgga agctcttgga tcaatgggaa atgatgcacc cctagcagtg atgtcaaaca 3300
gagagaagct gacttttgag tacttcaagc agatgtttgc acaagtaaca aaccctccaa 3360
tcgatccaat tagggagaag attgttacat ctatggaatg tatgattggg ccagaaggag 3420
atttgctgga aataaccgaa aagcaatgca accgccttgc acttaaaggt cctttggtgt 3480
caatggatga aatggaatct atcaagaaga tgaactaccg tggttggcgc agcaaggtgc 3540
tcgacataac ttatccgaag aattctggaa ggaagggctt ggaagaaact ttggatagaa 3600
tttgtgctga agcccgggaa gctatacgcg agggatacaa aattttagtt ctttcagaca 3660
gaggtcagtg acttattcac tttatacttg catcccattg tttgatggta caaattactc 3720
ataagttgac aaaaaattct agcagatggg tcaggtgtgc acaatttatt atcgttagct 3780
tccagcaaac atatcacatc accatatagt aacttaaata tgatcataat tttgtttgat 3840
agtcaattgt ttgcagtgta atgtgttgta tgaaggacat tcttactttg tcaccttgga 3900
cataattgga atgtatatta agtttattat gcaactcaac atcttccaat actgaaattt 3960
gttgtatttt cttgattcag gattttcttc agaccgtgtt gctgtcagtt ccctcttagc 4020
agttggagca gtacatcaac accttgttgc aaatcttgag aggacacgcg taggattatt 4080
ggttgagtct gctgaacctc gtgaagtgca ccatttctgt acactcgttg gatttggtgc 4140
agatgctata tgcccttatt tggccattga agcaatttgg tgcttgcaga ctgatgggaa 4200
gattcccccct accgactcaa aggaggaact tgtcgaaaaa tatttttatg cttccatcta 4260
tggaatgatg aaggttcttg caaagatggg aatatccacc cttgcatctt acaaaggggc 4320
acagattttt gaagctcttg gactttcttc tgaagtgatt cacaagtgtt ttgaaggcac 4380
tcctagcaga attgagggtg caacgttcga aatgcttgca cgtgatgctc tccgtcttca 4440
cgagttggca ttcccatcaa gaacacctcc gcctggcagt gcagatgcaa aagcccttcc 4500
caatccaggg gattaccact ggaggaaaaa tggtgaagtc catctaaatg atcctcttgc 4560
aatggcaaaa ttacaagaag cagctaaagt aaacagccgg gaagcataca aagaatattc 4620
taagcgaatt caagagctta acaaggcgtg caatctgcgt ggtatgctga aatttataga 4680
tagcactagc aagatttctt tggatgaggt tgaacctgca agtgagatag tgaaacgctt 4740
ttgtactggg gcaatgagtt atggatctat ttcgttggag gcacatactg cccttgctgt 4800
ggccatgaac aaactgggag gcaaatctaa cacaggtatt tcatgcatat gtgacatttt 4860

ttacccttg gctggttgtt acccttctga tgaaatcatg tatagtagct ttacttgcac 4920
atcaactctt agagccttgt tgcataacat atttttctta tttttgctgc agtagtgtag 4980
tgtctatgtt tctactgttt aaatatgccc tcgaagttgg tactttatac atgaccatgt 5040
ctagaaatgc actacttatt ttgatgctat cacatgaaaa ctatactctc atatttactt 5100
gtcgacatac ttgtactgca cttgtgtgtt tggttgcatt ctcgtctcag catagttccc 5160
tcttcatgca tgctcaactc aacacccctc ttcatgcatg ctttcttcat gcatgcttct 5220
agtgtatttg tgctaagcta gtgtggactc atgcaccctc catacactct accaaacacc 5280
caaaagtggg tcgagaaggg aactcttggg ctcatgcacc cttcatacac cctaccaaac 5340
acacccttaa tgcgctaagc ttttcatctt cctgttgatg ttgaatgcac ttctgtgaac 5400
aaattatccc agttcatagc aagagttaac ttctaagctt ttatctatct gttgatgttg 5460
aatgcacttc tggtagcaaa ttatcctaat tcctagcaag atttaacttc tgcttgcgta 5520
gaaacccaga cagcttgcac gcactattct tctttggact gtttgtatta gatattttct 5580
gcccacacag ttaatattat attgccaaat ctttgtattt attttcgctc ctaaactaaa 5640
ctcattttac ccatctgcta atttagggga gggaggggag cagccttctc gtatggagcc 5700
tcttcctgat ggttcgatga atccaaaacg aagtgcaatc aagcaagttg ccagtggacg 5760
atttggagtt tccagctatt atctgactaa tgcagatggg ctgcagataa aaatggctca 5820
ggtacttgac aatgctgtgc attgcgcaat cactggaatg atctcagttt atagaaagca 5880
actgtattca acgtcctgtg gttggcattt attctttttt tgtatcgtat atgtataggg 5940
tgctaagcct ggtgaaggtg gtgagcttcc aggtcacaag gttatcggtg acattgcagt 6000
taccagacat tctacagctg gtgttgggct tattagtcca cctcctcacc atgatatata 6060
ttctatcgag gatcttgcac agcttatcca tgaccttaag gtacattacc tgatgctctt 6120
ctatagccaa ggcatctgcc tatttatgta tggttgttag ttgctacttc tagattatat 6180
tggttatcta aacgctgtga tggtctaacc ctcctgagaa gctagaatca cttgctaaac 6240
tggtacaagt gatcctgcac atatgcatgt gtctccattt ggggggtttac cctccttttt 6300
gaaaaactca ttttgcaaat gttacctgct gatgtagtga tattcatgcc tgttttctct 6360
ctgcagaatt caaatcctca agctcgaatc agcgtgaagc tagtgtctga ggctggtgtt 6420
ggagttgtag ctagcggtgt tgtaaaagga cacgcagacc atgttcttat ttctggccat 6480
gacggtggca ccggtgcatc aagatggaca ggtatcaaga atgctggact tccatgggaa 6540
ctaggattgg ctgagacaca tcaaacttta gtggcaaatg ggcttcgtgg tcgagctgtc 6600
ctacaaacag atggccaatt aaaaactggt agagatgttg ctgtggcgtg cttacttggt 6660
gcagaggaat ttggtttcag cactgctcca ctgatcacac ttggctgcat tatgatgcgg 6720
aagtgccata ccaatacttg ccctgttggt atagccactc aagatccagt gctccgagaa 6780
aaatttgctg gagaaccaga gcatgtcatt aacttttttct tcatgcttgc tgaggagcta 6840
agagaaatta tggctcagct tggcctccgt acaattaatg aaatggttgg acgctctgat 6900
atgcttgaag ttgatccaga agtagttaag agcaatgaga aacttgaaaa tattgatctc 6960
tcattgatct taaaaccagc tgcagagatt cgtcctgggg ctgctcagta ctgtgttgaa 7020
aagcaagacc atggccttga catggctttg gataacaaac ttatagcttt atcaagggct 7080
gcacttgaaa aggaagttcg tgttttcatt gagactccaa tcaagaacac taatcgggca 7140

```
gtaggcacca cacttagcca tgaagtcaca aaacgttatc acatgaaggg attagatcct   7200
ggaaccattc atgtgaaact cactggaagt gctggtcaga gttttggtgc ttttctctgt   7260
cctggaataa cccttgagct tgaaggagac agcaatgatt acgttggcaa aggattatct   7320
ggtggaaaga ttgttgtgta cccacccagg aacagtacat ttagtgcaga agacaatatt   7380
gtcattggta atgtggccct gtatggtgct accaagggag aagcatactt caatggaatg   7440
gcagcagaaa ggttttgtgt tcgtaattct ggtgctcgaa cagtggttga aggaattggt   7500
gatcatggat gcgagtacat gacagggggt actgtagtca tccttggtaa aacaggaaga   7560
aattttgctg ctgggatgag tggaggcatc gcttatgttt atgatgttga tgggacattc   7620
agtgtccgct gtaacaatga gttggttgat ctatatcatg tggaggaaga ggatgatgta   7680
accactttga aaatgatgat agagcaacat cgacttcaca cagagagtgt cctggccaaa   7740
gacatactct ctaaattcga cactcttctt ccaaaatttg taaaagtata cccaagggat   7800
tataagaggg ttctagaaga aatgaaggca gaaaaagctg cagctaggcc tacaaaggaa   7860
cctaaggtgg caaatggtgt ttctgtgaca actaaggtaa tatatgattt aagtaataat   7920
tcttctgcat accttttta tttgaattta tagtagagat ggtgttctcc atttaggttt   7980
atccatcaga aattgcaatg atacacattg ccactactgt gtgaacttct atttttgcct   8040
aagtagatta actttgatct acagaaaata caaacagaga agtcatccag ccgaccaaca   8100
cgtgttgcca acgccaaaaa gtaccggggt tttgtaacat atgagcgaga gggtgtttct   8160
taccgtgatc caaatgagcg tgttaaagat tggaacgagg ttgccattga atcagttcca   8220
gggccactgt taaacacaca gtctgctcgt tgtatggatt gtggcactcc tttctgtcat   8280
caggtgaagc tgagttcatc ttttctattt aacattgatg attgtagttg gatttattgt   8340
ggggttaagg attgatatgt ttagtcattg actacatatg acattgagat gctaagattt   8400
catgattatt tgtacaggaa agctcaggtg ctggctgtcc tcttggaaat aagatcccag   8460
agttcaatga attagttcac cagaatagat ggcgtgaagc attggatcgc ctactagaga   8520
caaacaactt ccctgaattt actggacgtg tgtgccctgc tccttgtgag gggtcttgtg   8580
ttcttggcat tattgagaac ccagtatcaa tcaaaagcat agaatgctcg attatagaca   8640
aaggttttga agagggatgg atggtaccac gaccaccact tcaaagaaca gggtatgtct   8700
tttttaccat ctagttcggc ttattccctt atttatttac ttctgtttca ttaataggta   8760
acatttgagt tacactttct aatataaact gttcctttcg cagaaagaaa atcgctatag   8820
ttggcagtgg tcctgctggt ttggctgccg ctgatcaact aaataaaatg ggccattttg   8880
taactgtatt tgaacgttcg gatcgtatag gaggtcttat gatgtatgga gtaccaaaca   8940
tgaagacaga caagattgga gttgttcagc gtcgtgtcaa tttaatggcc gaagagggtg   9000
taacatttgt ggtgaatgct aatgtaggga gtgatccttt atactcaatt gaacgtctcc   9060
attctgagaa caatgcagtt attttggctt gtggagctac aaaaccaagg taactaattg   9120
gaacgaggat tttttttttc tagtttactc caagagtagc aacaatctca aaagaactga   9180
catatatctt ttgacactaa ctgactaaca tcatgtggga tattcttgaa cagggacctc   9240
agtattcctg gccgtgagct agctggagtt cattttgcca tggaatttct ccacgcaaat   9300
accaaaagtt tgcttgatag caacctggag gatggaagat acatatctgc ccagggtaag   9360
aaggtggtgg tcattggtgg tggagacaca ggcacagatt gcatcggtac gtctgttagg   9420
```

catggttgca gcagcattgt aaatctggag cttctcacca agccaccaag caagagagct 9480
tctgacaacc cctggcccca ggtaaatgca aaaaaaaaaa aactggatat ttccctgcac 9540
atgtgaattt ggccattcca tttccagctt ggaaagttct taagtcatca ttctgttttt 9600
gcatgctgtg gcctagagtc ttccgagtgg actatgggca ccaggaagca tctaccaaat 9660
ttggaaatga tccaagaact tactaagtct taaccaagcg tttcattggg tgatgaagat 9720
ggaaaattga aggcccttga ggtggtgcgc gtgaagtggg agaaagtaga tggaaaattc 9780
cagttcaagg agattgaagg atcacaagag atcatcgagg cagaccttgt cctccttgcc 9840
atgggattcc tgggccctga agaggttagt tatgcagact ctgaactact gtcacgcctg 9900
agttttatct tgatgataaa tgttggacaa acaactcacc gttttatttc tctgcagaac 9960
atcgctgaca aactgggttt agagaaagac aaccgttcca acttcaaagc tcaattcgga 10020
cacttcggga ccagtgtgga tggcgttttt gccgctgggg attgcaggcg cgggcaatcg 10080
ctggttgttt gggccatcac cgaagggcgt gaagctgctg ctgcagtaga taagtacttg 10140
tcaagggatg aacaaaatgt cgcaggcctt aca 10173

<210> 3
<211> 8964
<212> DNA
<213> Triticum aestivum
<220>
<221> misc_feature
<222> (913)..(913)
<223> n is a, c, g, or t
<220>
<221> misc_feature
<222> (959)..(959)
<223> n is a, c, g, or t
<220>
<221> misc_feature
<222> (1150)..(1150)
<223> n is a, c, g, or t
<220>
<221> misc_feature
<222> (2562)..(2562)
<223> n is a, c, g, or t
<220>
<221> misc_feature
<222> (3476)..(3476)
<223> n is a, c, g, or t

<220>
<221> misc_feature
<222> (5868)..(5868)
<223> n is a, c, g, or t
<220>
<221> misc_feature
<222> (5898)..(5898)
<223> n is a, c, g, or t
<220>
<221> misc_feature
<222> (5939)..(5939)
<223> n is a, c, g, or t
<400> 3
gaggaggctc tcaattgtat ctatccgggc cgcgctgaat cttcagcgtg gaggagagag 60
agatttctac atgtgctctc tatcttcaag gtccttactt cttctgaatt gcattttcac 120
tacaaactga tgaggttgag atcagggagc ccaattaagc tcctgacttt aatggaacat 180
taacaacaca aaagttgata ggaaagctga ctgggaaaaa caatcctggg ctatctctta 240
agcattagaa atctgtaatg taaggacatt tcatttttgc tgtattcatg tgagtaatta 300
ttgttaaatc ttctattgtg gttttccagg accattgttt acaagggcca acttatgcca 360
tcccagctcc aagggtacta ctatgcggat ataggtcatg aaaacttctc cagttatatg 420
gctctggtaa atacgaaacc ttgatactct tacaacaaaa gtcatggtcg tagtcagcgg 480
atgggaatca ataatagcaa gccagttgta agatcaagac tcgctcctgt gcagacatca 540
aatttgagct gtctattcat atgctatttc agtcctgctt cgtgatgcct ttaagtgaag 600
tactagaaac ttctcttagt tgatacccga taaccatgtt gcagagtcca gctgcagttc 660
tgctatggcc gactgcttgc cgactcttca ggatgtgacc gaagaaattg tgtttggttt 720
gaggtagctt agggctccca actgttttgc atgaaattat acgcagctga cgtattttta 780
caaaattcgt tcttcacaag gaagttttgt tttgaatatc tgcaaaagat gtttataata 840
tgatttctaa aagtcatatc ttgaaacagg ttcactcaag gttctccacc aacaccttcc 900
ccagctggga ccntgcacag ccaatgcgtg tcttaggcca caatggagag atcaatacnc 960
tcaaagggaa caaaaactgg taatattatt gtcaaatctt ttcctctttg ctttaacctt 1020
ttcttggcag atgcaactat ctcaatatgc acgttgtata tatggcagga tgaaagctcg 1080
tgagggtctc ttggagtgtg agaagcttgg cctatcacag gatgaaatgt caaagattct 1140
tccaatagtn gatgccactt cttcagattc aggtccgcag tctaatatgc agcatgaatt 1200
ccacttggca aataaaaatt catctcggtt ttgtgagtta ctaagtgtga aaaaacatgt 1260
gaattctagg tgcatttgat ggtgttctcg agctccttat ccgtggtgga agaagcctgc 1320
cagaagctgt gatgatgatg atccctgagg cgtggcagaa tgatgtaaac atggaacctg 1380
ataagaaagc tctgtacgag ttcttgtcag cccttatgga gccttgggat ggacctgctc 1440
tcatatcttg taaaaaactt aaacccgttt ctttttctg atcatacttc taactgatta 1500

cttaagctaa catgggtaca acctgtagtt accgatggcc gctaccttgg agctaccctg 1560
gatcgcaatg gccttaggcc tggtcgattt tatgtgaccc acagtggacg tgtggtcatg 1620
ggtagtgaag ttggtgttgt ggatattcct gcccaagatg tgttgagaaa gggtcggctt 1680
aaccctggaa tgatgttact cgttgacttc gacaaccata ctgtagtaga tgatgaagca 1740
ctcaaggcac agtactctaa agctcacccg tatggagaat ggctcaaacg acaaaagatg 1800
tacctcaaag acattgtaga atctgtccca gaaactgaca gagttgctcc aagcatttct 1860
ggttctatta cggtaagtac ttacgtggtc caacttcctc attcactggg tagtttggtg 1920
acgaaaattg gtaaaccata agtaacatgg tgattttaat attcttttac aatttggtgt 1980
agcaaacaaa tgagaacaag gaatgtgtag gcatcaatgc aattgtgact ccactaaagg 2040
cgtttgggta agcaaggatt aaacattgtc aactatcact catgtttcag tttctgctgg 2100
ctaatgatcc aaccgattaa ctacaggtac acattggaag ccctagaaat gttgctgctg 2160
ccaatggcta aagatggagt ggaagctctt ggatcaatgg gaaatgatgc acccctagca 2220
gtgatgtcaa acagagagaa gctgactttt gagtacttca agcagatgtt tgcacaagta 2280
acaaaccctc caatcgatcc aattagggag aagattgtta catctatgga atgtatgatt 2340
gggccagaag gagatttgct ggaaataacc gaaaagcaat gcaaccgcct tgcacttaaa 2400
ggtcctttgg tgtcaatgga tgaaatggaa tctatcaaga agatgaacta ccgtggttgg 2460
cgcagcaagg tgctcgacat aacttatccg aagaattctg gaaggaaggg cttggaagaa 2520
actttggata gaatttgtgc tgaagcccgg gaagctatac gngagggata caaaatttta 2580
gttctttcag acagaggtca gtgacttatt cactttatac ttgcatccca ttgtttgatg 2640
gtacaaatta ctcataagtt gacaaaaaat tctagcagat gggtcaggtg tgcacaattt 2700
attatcgtta gcttccagca aacatatcac atcaccatat agtaacttaa atatgatcat 2760
aattttgttt gatagtcaat tgtttgcagt gtaatgtgtt gtatgaagga cattcttact 2820
ttgtcacctt ggacataatt ggaatgtata ttaagtttat tatgcaactc aacatcttcc 2880
aatactgaaa tttgttgtat tttcttgatt caggattttc ttcagaccgt gttgctgtca 2940
gttccctctt agcagttgga gcagtacatc aacaccttgt tgcaaatctt gagaggacac 3000
gcgtaggatt attggttgag tctgctgaac ctcgtgaagt gcaccatttc tgtacactcg 3060
ttggatttgg tgcagatgct atatgccctt atttggccat tgaagcaatt tggtgcttgc 3120
agactgatgg gaagattccc cctaccgact caaaggagga acttgtcgaa aaatattttt 3180
atgcttccat ctatggaatg atgaaggttc ttgcaaagat gggaatatcc acccttgcat 3240
cttacaaagg ggcacagatt tttgaagctc ttggactttc ttctgaagtg attcacaagt 3300
gttttgaagg cactcctagc agaattgagg gtgcaacgtt cgaaatgctt gcacgtgatg 3360
ctctccgtct tcacgagttg gcattcccat caagaacacc tccgcctggc agtgcagatg 3420
caaaagccct tcccaatcca ggggattacc actggaggaa aaatggtgaa gtccanctaa 3480
atgatcctct tgcaatggca aaattacaag aagcagctaa agtaaacagc cgggaagcat 3540
acaaagaata ttctaagcga attcaagagc ttaacaaggc gtgcaatctg cgtggtatgc 3600
tgaaatttat agatagcact agcaagattt ctttggatga ggttgaacct gcaagtgaga 3660
tagtgaaacg cttttgtact ggggcaatga gttatggatc tatttcgttg gaggcacata 3720
ctgcccttgc tgtggccatg aacaaactgg gaggcaaatc taacacaggt atttcatgca 3780

tatgtgacat tttttaccct ttcgctggtt gttacccttc tgatgaaatc atgtatagta 3840
gctttacttg cacatcaact cttagagcct tgttgcataa catatttttc ttatttttgc 3900
tgcagtagtg tagtgtctat gtttctactg tttaaatatg ccctcgaagt tggtacttta 3960
tacatgacca tgtctagaaa tgcactactt attttgatgc tatcacatga aaactatact 4020
ctcatagcag tacaagtatt tatcttcctg ttgatgttga atgcacttct gtgaaaacta 4080
tactctcata gtagtgtcta tgtttctaca agtatttatc acatgaaaac tatactcaca 4140
taaaaccatt tcatcttcct gttgatgttg aatgcacttc tgtgaacaaa ttatcccagt 4200
tcatagcaag agttaacttc taagctttta tctatctgtt gatgttgaat gcacttctgg 4260
tagcaaatta tcctaattcc tagcaagatt taacttctgc ttgcgtagaa acccagacag 4320
cttgcacgca ttattcttct ttggactgtt tgtattatag atattttctg cccacacagt 4380
taatattata ttgccaaatc tttgtattta ttttcactcc taaactaaac tcattttacc 4440
catctgctaa tttaggggag ggaggggagc agccttctcg tatggagcct cttcctgatg 4500
gttcgatgaa tccaaaacga agtgcaatca agcaagttgc cagtggacga tttggagttt 4560
ccagctatta tctgactaat gcagatgagc tgcagataaa aatggctcag gtacttgaca 4620
atgctgtgca ttgcgcaatc actggaatga tctcagttta tagaaagcaa ctgtattcaa 4680
cgtcctgtgg ttggcattta ttcttttttt gtatcgtata tgtatagggt gctaagcctg 4740
gtgaaggtgg tgagcttcca ggtcacaagg ttatcggtga cattgcagtt accagacatt 4800
ctacagctgg tgttgggctt attagtccac ctcctcacca tgatatatat tctatcgagg 4860
atcttgcaca gcttatccat gaccttaagg tacattacct gatgctcttc tatagccaag 4920
gcatctgcct atttatgtat ggttgttagt tgctacttct agattatatt ggttatctaa 4980
acgctgtgat ggtctaaccc tcctgagaag ctagaatcac ttgctaaact ggtacaagtg 5040
atcctgcaca tatgcatgtg tctccatttg gggtttaccc ctcctttttg aaaaactcat 5100
tttgcaaatg ttacctgctg atgtagtgat attcatgcct gttttctctc tgcagaattc 5160
aaatcctcaa gctcgaatca gcgtgaagct agtgtctgag gctggtgttg gagttgtagc 5220
tagcggtgtt gtaaaaggac acgcagacca tgttcttatt tctggccatg acggtggcac 5280
cggtgcatca agatggacag gtatcaagaa tgctggactt ccatggggaa ctaggattgg 5340
ctgagacaca tcaaacttta gtggcaaatg ggcttcgtgg tcgagctgtc ctacaaacag 5400
atggccaatt aaaaactggt agagatgttg ctgtggcgtg cttacttggt gcagaggaat 5460
ttggtttcag cactgctcca ctgatcacac ttggctgcat tatgatgcgg aagtgccata 5520
ccaatacttg ccctgttggt atagccactc aagatccagt gctccgagaa aaatttgctg 5580
gagaaccaga gcatgtcatt aacttttttct tcatgcttgc tgaggagcta agagaaatta 5640
tggctcagct tggcctccgt acaattaatg aaatggttgg acgctctgat atgcttgaag 5700
ttgatccaga agtagttaag agcaatgaga aacttgaaaa tattgatctc tcattgatct 5760
taaaaccagc tgcagagatt cgtcctgggg ctgctcagta ctgtgttgaa aagcaagacc 5820
atggccttga catggctttg gataacaaac ttatagcttt atcaaggnct gcacttgaaa 5880
aggaagttcg tgttttcntt gagactccaa tcaagaacac taatcgggca gtaggcacna 5940
cacttagcca tgaagtcaca aaacgttatc acatgaaggg attagatcct ggaaccattc 6000
atgtgaaact cactggaagt gctggtcaga gttttggtgc ttttctctgt cctggaataa 6060

cccttgagct tgaaggagac agcaatgatt acgttggcaa aggattatct ggtggaaaga 6120
ttgttgtgta cccacccagg aacagtacat ttagtgcaga agacaatatt gtcattggta 6180
atgtggccct gtatggtgct accaagggag aagcatactt caatggaatg gcagcagaaa 6240
ggttttgtgt tcgtaattct ggtgctcgga cagtggttga aggaattggt gatcatggat 6300
gcgagtacat gacagggggt actgtagtca tccttggtaa aacaggaaga aattttgctg 6360
ctgggatgag tggaggcatc gcttatgttt atgatgttga tgggacattc agtgtccgct 6420
gtaacaatga gttggttgat ctatatcatg tggaggaaga ggatgatgta accactttga 6480
aaatgatgat agagcaacat cgacttcaca cagagagtgt cctggccaaa gacatactct 6540
ctaaattcga cactcttctt ccaaaatttg taaaagtata cccaagggat tataagaggg 6600
ttctagaaga aatgaaggca gaaaaagctg cagctaggcc tacaaaggaa cctaaggtgg 6660
caaatggtgt ttctgtgaca actaaggtaa tatatgattt aagtaataat tcttctgcat 6720
acctttttaa tttgaattta tagtagagat ggtgttctcc atttaggttt atccatcaga 6780
aattgcaatg atacacattg ccactactgt gtgaacttct atttttgcct aagtagatta 6840
actttgatct acagaaaata caaacagaga agtcatccag ccgaccaaca cgtgttgcca 6900
acgccaaaaa gtaccggggt tttgtaacat atgagcgaga gggtgtttct taccgtgatc 6960
caaatgagcg tgttaaagat tggaacgagg ttgccattga atcagttcca gggccactgt 7020
taaacacaca gtctgctcgt tgtatggatt gtggcactcc tttctgtcat caggtgaagc 7080
tgagttcatc ttttctattt aacattgatg attgtagttg gatttattgt ggggttaagg 7140
attgatatgt ttagtcattg gctacatatg acattgagat gctaagattt catgattatt 7200
tgtacaggaa agctcaggtg ctggctgtcc tcttggaaat aagatcccag agttcaatga 7260
attagttcac cagaatagat ggcgtgaagc attggatcgc ctactagaga caaacaactt 7320
ccctgaattt actggacgtg tgtgccctgc tccttgtgag ggtcttgtg ttcttggcat 7380
tattgagaac ccagtatcaa tcaaaagcat agaatgctcg attatagaca aaggttttga 7440
agagggatgg atggtaccac gaccaccact tcaaagaaca gggtatgtct tttttaccat 7500
ctagttcggc ttattccctt atttatttac ttctgtttca ttaataggta acatttgagt 7560
tacactttct aatataaact gttcctttcg cagaaagaaa atcgctatag ttggcagtgg 7620
tcctgctggt ttggctgccg ctgatcaact aaataaaatg ggccattttg taactgtatt 7680
tgaacgttcg gatcgtatag gaggtcttat gatgtatgga gtaccaaaca tgaagacaga 7740
caagattgga gttgttcagc gtcgtgtcaa tttaatggcc gaagagggta taacatttgt 7800
ggtgaatgct aatgtaggga gtgatccttt atactcaatt gaacgtctcc gttctgagaa 7860
caatgcagtt attttggctt gtggagctac aaaaccaagg taactaattg gaacgaggat 7920
tttttttttc tagtttactc caagagtagc aacaatctca aaagaactga catatatctt 7980
ttgacactaa ctgactaaca tcatgtggga tattcttgaa cagggacctc agtattcctg 8040
gccgtgagct agctgcagtt cattttgcca tggaatttct ccacgcaaat accaaaagtt 8100
tgcttgatag caacctggag gatggaagat acatatctgc ccagggtaag aaggtggtgg 8160
tcattggtgg tggagacaca ggcacagatt gcatcggtac gtctgttagg catggttgca 8220
gcagcattgt aaatctggag cttctcacca agccaccaag caagagagct tctgacaacc 8280
cctggcccca ggtaaatgca aaaaaaaaaa actggatatt tccctgcaca tgtgaatttg 8340

gccattccat ttccagcttg gaaagttctt aagtcatcat tctgtttttg catgcagtgg 8400
cctagagtct tccgagtgga ctatgggcac caggaagcat ctaccaagtt tggaaatgat 8460
ccaagaactt acgaagtctt aaccaagcgt ttcattggtg atgaagatgg aaaattgaag 8520
gcccttgagg tggtgcgcgt gaagtgggag aaagtagatg gaaaattcca gttcaaggag 8580
attgaaggat cacaagagat catcgaggca gaccttgtcc tccttgccat gggattcctg 8640
ggccctgaag aggttagtta tgcagactct gaactactgt cacgcctgag ttttatcttg 8700
atgataaatg ttggacaaac aactcaccgt tttatttctc tgcagaacat cgctgacaaa 8760
ctgggtttag agaaagacaa ccgttccaac ttcaaagctc aattcggaca cttcgggacc 8820
agtgtggatg gcgtttttgc cgctggggat tgcaggcgcg ggcaatcgct ggttgtttgg 8880
gccatcaccg aagggcgtga agctgctgct gcagtagata agtacttgtc aagggatgaa 8940
caaaatgtcg caggccttac atag 8964

<210> 4
<211> 9113
<212> DNA
<213> Triticum aestivum
<220>
<221> misc_feature
<222> (1140)..(1140)
<223> n is a, c, g, or t
<220>
<221> misc_feature
<222> (1155)..(1155)
<223> n is a, c, g, or t
<220>
<221> misc_feature
<222> (1220)..(1220)
<223> n is a, c, g, or t
<220>
<221> misc_feature
<222> (1239)..(1239)
<223> n is a, c, g, or t
<220>
<221> misc_feature
<222> (1294)..(1294)
<223> n is a, c, g, or t
<220>
<221> misc_feature

<222> (1351)..(1351)
<223> n is a, c, g, or t
<220>
<221> misc_feature
<222> (2648)..(2648)
<223> n is a, c, g, or t
<220>
<221> misc_feature
<222> (2747)..(2747)
<223> n is a, c, g, or t
<220>
<221> misc_feature
<222> (2772)..(2772)
<223> n is a, c, g, or t
<220>
<221> misc_feature
<222> (2791)..(2791)
<223> n is a, c, g, or t
<220>
<221> misc_feature
<222> (4426)..(4426)
<223> n is a, c, g, or t
<220>
<221> misc_feature
<222> (4431)..(4431)
<223> n is a, c, g, or t
<220>
<221> misc_feature
<222> (6016)..(6016)
<223> n is a, c, g, or t
<220>
<221> misc_feature
<222> (6046)..(6046)
<223> n is a, c, g, or t
<220>
<221> misc_feature
<222> (6087)..(6087)
<223> n is a, c, g, or t

<220>
<221> misc_feature
<222> (7938)..(7938)
<223> n is a, c, g, or t
<220>
<221> misc_feature
<222> (7962)..(7962)
<223> n is a, c, g, or t
<220>
<221> misc_feature
<222> (8118)..(8118)
<223> n is a, c, g, or t
<220>
<221> misc_feature
<222> (8190)..(8190)
<223> n is a, c, g, or t
<400> 4

```
atcctgagga ggctctcaat tgtatctatc cgggccgcgc tgaatcttca gcgtggagga     60
gagagagatt tctacatgtg ctctctatct tcaaggtcct tacttcttct gaattgcatt    120
ttcactacaa actgatgagg ttgagatcag ggagcccaat taagctcctg actttaatgg    180
aacattaaca acacaaaagt tgataggaaa gctgactggg aaaaacaatc ctgggctatc    240
tcttaagcat tagaaatctg taatgtaagg acatttcatt tttgctgtat tcatgtgagt    300
aattattgtt aaatcttcta ttgtggtttt ccaggaccat tgtttacaag ggccaactta    360
tgccatccca gctccaaggg tactactatg cggatatagg tcatgaaaac ttctccagtt    420
atatggctct ggtaaatacg aaaccttgat actcttacaa caaaagtcat ggtcgtagtc    480
agcggatggg aatcaataat agcaagccag ttgtaagatc aagactcgct cctgtgcaga    540
catcaaattt gagctgtcta ttcatatgct atttcagtcc tgcttcgtga tgcctttaag    600
tgaagtacta gaaacttctc ttagttgata cccgataacc atgttgcaga gtccagctgc    660
agttctgcta tggccgactg cttgccgact cttcaggatg tgaccgaaga aattgtgttt    720
ggtttgaggt agcttagggc tcccaactgt tttgcatgaa attatacgca gctgacgtat    780
ttttacaaaa ttcgttcttc acaaggaagt tttgttttga atatctgcaa aagatgttta    840
taatatgatt tctaaaagtc atatcttgaa acaggttcac tcaaggttct ccaccaacac    900
cttccccagc tgggaccgtg cacagccaat gcgtgtctta ggccacaatg gagagatcaa    960
tactctcaaa gggaacaaaa actggtaata ttattgtcaa atcttttcct ctttgcttta   1020
accttttctt ggcagatgca actatctcaa tatgcacgtt gtatatatgg caggatgaaa   1080
gctcgtgagg gtctcttgga gtgtgagaag cttggcctat cacaggatga aatgtcaaan   1140
attcttccaa tagtngatgc cacttcttca gattcaggtc cgcagtctaa tatgcagcat   1200
gaattccact tggcaaatan aaattcatct cggttttgng agttactaag tgtgaaaaaa   1260
```

```
catgtgaatt ctaggtgcat ttgatggtgt tctngagctc cttatccgtg gtggaagaag   1320
cctgccagaa gctgtgatga tgatgatccc ngaggcgtgg cagaatgatg taaacatgga   1380
acctgataag aaagctctgt acgagttctt gtcagccctt atggagcctt gggatggacc   1440
tgctctcata tcttgtaaaa aacttaaacc cgtttctttt ttctgatcat acttctaact   1500
gattacttaa gctaacatgg gtacaacctg tagttaccga tggccgctac cttggagcta   1560
ccctggatcg caatggcctt aggcctggtc gattttatgt aacccacagt ggacgtgtgg   1620
tcatgggtag tgaagttggt gttgtggata ttcctgccca agatgtgttg agaaagggtc   1680
ggcttaaccc tggaatgatg ttactcgttg acttcgacga ccatactgta gtagatgatg   1740
aagcactcaa ggcacagtac tctaaagctc acccgtatgg agaatggctc aagcgacaaa   1800
agatgtacct caaagacatt gtagaatctg tcccagaaac tgacagagtt gctccaagca   1860
tttctggttc tattacggta agtacttacg tggtccaact tcctcattca ctgggtagtt   1920
tggtgatgaa aattggtaaa ccataaggaa catggtgatt ttaatattct tttacaattt   1980
ggtgtagcaa acaaatgaga acaaggaatg tgtaggcatc aatgcaattg tgactccact   2040
aaaggcgttt gggtaagcaa ggattgaaca ttgtcaacta tcactcatgt ttcaatttct   2100
gctggctaat gatccaaccg attaactaca ggtacacatt ggaagcccta gaaatgttgc   2160
tgctgccaat ggctaaagat ggagtggaag ctcttggatc aatgggaaat gatgcacccc   2220
tagcagtgat gtcaaacaga gagaagctga cttttgagta cttcaagcag atgtttgcac   2280
aagtaacaaa ccctccaatc gatccaatta gggagaagat tgttacatct atggaatgta   2340
tgattgggcc agaaggagat ttgctggaaa taaccgaaaa gcaatgcaac cgccttgcac   2400
ttaaaggtcc tttggtgtca atggatgaaa tggaatctat caagaagatg aactaccgtg   2460
gttggcgcag caaggtgctc gacataactt atccgaagaa ttctggaagg aaggggttgg   2520
aagaaacttt ggatagaatt tgtgctgaag cccgggaagc tatacgcaag ggatacaaaa   2580
ttttagttct ttcagacaga ggtcagtgac ttattcactt tatacttgca tcccattgtt   2640
tgatggtnca aattactcat aagttgacaa aaaattctag cagatgggtc aggtgtgcac   2700
aatttattat cgttagcttc cagcaaacat atcacatcac catatantaa cttaaatatg   2760
atcataattt tntttgatag tcaattgttt ncagtgtaat gtgttgtatg aaggacattc   2820
ttactttgtc accttggaca taattggaat gtatattaag tttattatgc aactcaacat   2880
cttccaatac tgaaatttgt tgtattttct tgattcagga ttttcttcag accgtgttgc   2940
tgtcagttcc ctcttagcag ttggagcagt acatcaacac cttgttgcaa atcttgagag   3000
gacacgcgta ggattattgg ttgagtctgc tgaacctcgt gaagtgcacc atttctgtac   3060
actcgttgga tttggtgcag atgctatatg ccccttattg gccattgaag caatttggtg   3120
cttgcagact gatgggaaga ttccccctac cgactcaaag gaggaacttg tcgaaaaata   3180
tttttatgct tccatctatg gaatgatgaa ggttcttgca aagatgggaa tatccaccct   3240
tgcatcttac aaaggggcac agatttttga agctcttgga ctttcttctg aagtgattca   3300
caagtgtttt gaaggcactc ctagcagaat tgagggtgca acgttcgaaa tgcttgcacg   3360
tgatgctctc cgtcttcacg agttggcatt cccatcaaga acacctccgc ctggcagtgc   3420
agatgcaaaa gcccttccca atccagggga ttaccactgg aggaaaaatg gtgaagtcca   3480
tctaaatgat cctcttgcaa tggcaaaatt acaagaagca gctaaagtaa acagccggga   3540
```

agcatacaaa gaatattcta agcgaattca agagcttaac aaggcgtgca atctgcgtgg 3600
tatgctgaaa tttatagata gcactagcaa gatttctttg gatgaggttg aacctgcaag 3660
tgagatagtg aaacgctttt gtactggggc aatgagttat ggatctattt cgttggaggc 3720
acatactgcc cttgctgtgg ccatgaacaa actgggaggc aaatctaaca caggtatttc 3780
atgcatatgt gacatttttt accctttggc tggttgttac ccttctgatg aaatcatgta 3840
tagtagcttt acttgcacat caactcttag agccttgttg cataacatat ttttcttatt 3900
tttgctgcag tagtgtagtg tctatgtttc tactgtttaa atatgccctc gaagttggta 3960
ctttatacat gaccatgtct agaaatgcac tacttatttt gatgctatca catgaaaact 4020
atactctcat atttacttgt ggacatactt gtactgcact tgtgtgtttg gttgcattct 4080
cgtctcagca tagttccctc ttcatgcatg ctcaactcaa cacccctctt catgcatgct 4140
ttcttcatgc atgcttctag tgtatttgtg ctaagctagt gtggactcat gcaccctcca 4200
tacactctac caaacaccca aaagtgggtc gagaagggaa ctcttgggct catgcaccct 4260
tcatacaccc taccaaacac acccttaatg cgctaagctt ttcatcttcc tgttgatgtt 4320
gaatgcactt ctgtgaacaa attatcccag ttcatagcaa gagttaactt ctaagctttt 4380
atctatctgt tgatgttgaa tgcacttctg gtagcaaatt atcctnattc ntagcaagat 4440
ttaacttctg cttgcgtaga aacccagaca gcttgcacgc actattcttc tttggactgt 4500
ttgtattaga tattttctgc ccacacagtt aatattatat tgccaaatct ttgtatttat 4560
tttcgctcct aaactaaact cattttaccc atctgctaat ttagggggagg gaggggagca 4620
gccttctcgt atggagcctc ttcctgatgg ttcgatgaat ccaaaacgaa gtgcaatcaa 4680
gcaagttgcc agtggacgat ttggagtttc cagctattat ctgactaatg cagatgagct 4740
gcagataaaa atggctcagg tacttgacaa tgctgtgcat tgcgcaatca ctggaatgat 4800
ctcagtttat agaaagcaac tgtattcaac gtcctgtggt tggcatttat tctttttttg 4860
tatcgtatat gtatagggtg ctaagcctgg tgaaggtggt gagcttccag gtcacaaggt 4920
tatcggtgac attgcagtta ccagacattc tacagctggt gttgggctta ttagtccacc 4980
tcctcaccat gatatatatt ctatcgagga tcttgcacag cttatccatg accttaaggt 5040
acattacctg atgctcttct atagccaagg catctgccta tttatgtatg gttgttagtt 5100
gctacttcta gattatattg gttatctaaa cgctgtgatg gtctaaccct cctgagaagc 5160
tagaatcact tgctaaactg gtacaagtga tcctgcacat atgcatgtgt ctccatttgg 5220
gggtttaccc tcctttttga aaaactcatt ttgcaaatgt tacctgctga tgtagtgata 5280
ttcatgcctg ttttctctct gcagaattca aatcctcaag ctcgaatcag cgtgaagcta 5340
gtgtctgagg ctggtgttgg agttgtagct agcggtgttg taaaaggaca cgcagaccat 5400
gttcttattt ctggccatga cggtggcacc ggtgcatcaa gatggacagg tatcaagaat 5460
gctggacttc catggggaac taggattggc tgagacacat caaactttag tggcaaatgg 5520
gcttcgtggt cgagctgtcc tacaaacaga tggccaatta aaaactggta gagatgttgc 5580
tgtggcgtgc ttacttggtg cagaggaatt tggtttcagc actgctccac tgatcacact 5640
tggctgcatt atgatgcgga agtgccatac caatacttgc cctgttggta tagccactca 5700
agatccagtg ctccgagaaa aatttgctgg agaaccagag catgtcatta actttttctt 5760
catgcttgct gaggagctaa gagaaattat ggctcagctt ggcctccgta caattaatga 5820

aatggttgga cgctctgata tgcttgaagt tgatccagaa gtagttaaga gcaatgagaa 5880
acttgaaaat attgatctct cattgatctt aaaaccagct gcagagattc gtcctggggc 5940
tgctcagtac tgtgttgaaa agcaagacca tgccttgaca tggctttgga taacaaactt 6000
atagctttat caaggnctgc acttgaaaag gaagttcgtg ttttcnttga gactccaatc 6060
aagaacacta atcgggcagt aggcacnaca cttagccatg aagtcacaaa acgttatcac 6120
atgaagggat tagatcctgg aaccattcat gtgaaactca ctggaagtgc tggtcagagt 6180
tttggtgctt ttctctgtcc tggaataacc cttgagcttg aaggagacag caatgattac 6240
gttggcaaag gattatctgg tggaaagatt gttgtgtacc cacccaggaa cagtacattt 6300
agtgcagaag acaatattgt cattggtaat gtggccctgt atggtgctac caagggagaa 6360
gcatacttca atggaatggc agcagaaagg ttttgtgttc gtaattctgg tgctcgaaca 6420
gtggttgaag gaattggtga tcatggatgc gagtacatga caggggtac tgtagtcatc 6480
cttggtaaaa caggaagaaa ttttgctgct gggatgagtg gaggcatcgc ttatgtttat 6540
gatgttgatg ggacattcag tgtccgctgt aacaatgagt tggttgatct atatcatgtg 6600
gaggaagagg atgatgtaac cactttgaaa atgatgatag agcaacatcg acttcacaca 6660
gagagtgtcc tggccaaaga catactctct aaattcgaca ctcttcttcc aaaatttgta 6720
aaagtatacc caagggatta taagaggggtt ctagaagaaa tgaaggcaga aaaagctgca 6780
gctaggccta caaaggaacc taaggtggca aatggtgttt ctgtgacaac taaggtaata 6840
tatgatttaa gtaataattc ttctgcatac ctttttaatt tgaatttata gtagagatgg 6900
tgttctccat ttaggtttat ccatcagaaa ttgcaatgat acacattgcc actactgtgt 6960
gaacttctat ttttgcctaa gtagattaac tttgatctac agaaaataca aacagagaag 7020
tcatccagcc gaccaacacg tgttgccaac gccaaaaagt accggggttt tgtaacatat 7080
gagcgagagg gtgtttctta ccgtgatcca aatgagcgtg ttaaagattg gaacgaggtt 7140
gccattgaat cagttccagg gccactgtta aacacacagt ctgctcgttg tatggattgt 7200
ggcactcctt tctgtcatca ggtgaagctg agttcatctt ttctatttaa cattgatgat 7260
tgtagttgga tttattgtgg ggttaaggat tgatatgttt agtcattggc tacatatgac 7320
attgagatgc taagatttca tgattatttg tacaggaaag ctcaggtgct ggctgtcctc 7380
ttggaaataa gatcccagag ttcaatgaat tagttcacca gaatagatgg cgtgaagcat 7440
tggatcgcct actagagaca aacaacttcc ctgaatttac tggacgtgtg tgccctgctc 7500
cttgtgaggg gtcttgtgtt cttggcatta ttgagaaccc agtatcaatc aaaagcatag 7560
aatgctcgat tatagacaaa ggttttgaag agggatggat ggtaccacga ccaccacttc 7620
aaagaacagg gtatgtcttt tttaccatct agttcggctt attcccttat ttatttactt 7680
ctgtttcatt aataggtaac atttgagtta cactttctaa tataaactgt tcctttcgca 7740
gaaagaaaat cgctatagtt ggcagtggtc ctgctggttt ggctgccgct gatcaactaa 7800
ataaaatggg ccattttgta actgtatttg aacgttcgga tcgtatagga ggtcttatga 7860
tgtatggagt accaaacatg aagacagaca agattggagt tgttcagcgt cgtgtcaatt 7920
taatggccga agagggtnta acatttgtgg tgaatgctaa tntagggagt gatcctttat 7980
actcaattga acgtctccgt tctgagaaca atgcagttat tttggcttgt ggagctacaa 8040
aaccaaggta actaattgga acgaggattt ttttttcta gtttactcca agagtagcaa 8100

caatctcaaa agaactgnca tatatctttt gacactaact gactaacatc atgtgggata 8160
ttcttgaaca gggacctcag tattcctggn cgtgagctag ctggagttca ttttgccatg 8220
gaatttctcc acgcaaatac caaaagtttg cttgatagca acctggagga tggaagatac 8280
atatctgccc agggtaagaa ggtggtggtc attggtggtg gagacacagg cacagattgc 8340
atcggtacgt ctgttaggca tggttgcagc agcattgtaa atctggagct tctcaccaag 8400
ccaccaagca agagagcttc tgacaacccc tggccccagg taaatgcaaa aaaaaaaaaa 8460
ctggatattt ccctgcacat gtgaatttgg ccattccatt tccagcttgg aaagttctta 8520
agtcatcatt ctgtttttgc atgcagtggc ctagagtctt ccgagtggac tatgggcacc 8580
aggaagcatc taccaagttt ggaaatgatc caagaactta cgaagtctta accaagcgtt 8640
tcattggtga tgaagatgga aaattgaagg cccttgaggt ggtgcgcgtg aagtgggaga 8700
aagtagatgg aaaattccag ttcaaggaga ttgaaggatc acaagagatc atcgaggcag 8760
accttgtcct ccttgccatg ggattcctgg gccctgaaga ggttagttat gcagactctg 8820
aactactgtc acgcctgagt tttatcttga tgataaatgt tggacaaaca actcaccgtt 8880
ttatttctct gcagaacatc gctgacaaac tgggtttaga gaaagacaac cgttccaact 8940
tcaaagctca attcggacac ttcgggacca gtgtggatgg cgtttttgcc gctggggatt 9000
gcaggcgcgg gcaatcgctg gttgtttggg ccatcaccga agggcgtgaa gctgctgctg 9060
cagtagataa gtacttgtca agggatgaac aaaatgtcgc aggccttaca tag 9113

<210> 5
<211> 6432
<212> DNA
<213> Triticum aestivum

<400> 5
atgccgacgg cgcaggggga ttggtttgaa gccgcggcgc cgccgggcgg ccgcagggcc 60
cgccgcagcc agtctgcctc ggcgccgtgc cgctccgcaa ggcaggcgca cggcgccatg 120
tccctggatg gcgggttcct cggcggcgcg cagcgcaccg aggaacgcgt cgcgccacgc 180
ccgcctcggg ccgcggcgcg cgacgccgag tccatcaggc ccatgtctct gctacccgag 240
agcagcattg ggctgtacga cccggcgttc gagcgtgact cgtgcggcgt tggcttcgtc 300
gccgagctgt cgggcgttga caaccgggcg accgtcgtcg atgccattca gatgcttgaa 360
agaatggcac accgaggtgc ctgcggctgt gagaaaaaca ctggtgatgg tgccggcatt 420
ctcgttgctc taccacacac cttcttccga gaggtgacaa aggatgccgg tttcgagtta 480
ccgccaccag gtgagtatgc tgttggaatg gtcttcctgc caaccgatga gaagcgccgc 540
gagaggagca aaactgagtt tacaaaggtc gctgagtctc taggacattc gatacttggg 600
tggcgccagg ttcccactga caattcagac ttgggccaag ctgcgctcga tactgaacca 660
gccattgaac aggttttcct caccaagagt tcaaaatcga aggccgactt cgaacagcag 720
gtgtttatcc tgaggaggct ctcaattgta tctatccggg ccgcgctgaa tcttcagcgt 780
ggaggagaga gagatttcta catgtgctct ctatcttcaa ggaccattgt ttacaagggc 840
caacttatgc catcccagct ccaagggtac tactatgcgg atataggtca tgaaaacttc 900

tccagttata tggctctggt tcactcaagg ttctccacca acaccttccc cagctgggac 960
cgtgcacagc caatgcgtgt cttaggccac aatggagaga tcaatactct caaagggaac 1020
aaaaactgga tgaaagctcg tgagggtctc ttggagtgtg agaagcttgg cctatcacag 1080
gatgaaatgt caaagattct tccaatagta gatgccactt cttcagattc aggtgcattt 1140
gatggtgttc tcgagctcct tatccgtggt ggaagaagcc tgccagaagc tgtgatgatg 1200
atgatccctg aggcgtggca gaatgatgta aacatggaac ctgataagaa agctctgtac 1260
gagttcttgt cagcccttat ggagccttgg gatggacctg ctctcatatc ttttaccgat 1320
ggccgctacc ttggagctac cctggatcgc aatggcctta ggcctggtcg atttatgta 1380
acccacagtg gacgtgtggt catgggtagt gaagttggtg ttgtggatat tcctgcccaa 1440
gatgtgttga gaaagggtcg gcttaaccct ggaatgatgt tactcgttga cttcgacaac 1500
catactgtag tagatgatga agcactcaag gcacagtact ctaaagctca cccgtatgga 1560
gaatggctca agcgacaaaa gatgtacctc aaagacattg tagaatctgt cccagaaact 1620
gacagagttg ctccaagcat ttctggttct attacgcaaa caaatgagaa caaggaatgt 1680
gtaggcatca atgcaattgt gactccacta aaggcgtttg ggtacacatt ggaagcccta 1740
gaaatgttgc tgctgccaat ggctaaagat ggagtggaag ctcttggatc aatgggaaat 1800
gatgcacccc tagcagtgat gtcaaacaga gagaagctga cttttgagta cttcaagcag 1860
atgtttgcac aagtaacaaa ccctccaatc gatccaatta gggagaagat tgttacatct 1920
atggaatgta tgattgggcc agaaggagat ttgctggaaa taaccgaaaa gcaatgcaac 1980
cgccttgcac ttaaaggtcc tttggtgtca atggatgaaa tggaatctat caagaagatg 2040
aactaccgtg gttggcgcag caaggtgctc gacataactt atccgaagaa ttctggaagg 2100
aagggcttgg aagaaacttt ggatagaatt tgtgctgaag cccgggaagc tatacgcgag 2160
ggatacaaaa ttttagttct ttcagacaga ggattttctt cagaccgtgt tgctgtcagt 2220
tccctcttag cagttggagc agtacatcaa caccttgttg caaatcttga gaggacacgc 2280
gtaggattat tggttgagtc tgctgaacct cgtgaagtgc accatttctg tacactcgtt 2340
ggatttggtg cagatgctat atgcccttat ttggccattg aagcaatttg gtgcttgcag 2400
actgatggga agattccccc taccgactca aaggacgaac ttgtcgaaaa atatttttat 2460
gcttccatct atggaatgat gaaggttctt gcaaagatgg gaatatccac ccttgcatct 2520
tacaaagggg cacagatttt tgaagctctt ggactttctt ctgaagtgat tcacaagtgt 2580
tttgaaggca ctcctagcag aattgagggt gcaacgttcg aaatgcttgc acgtgatgct 2640
ctccgtcttc acgagttggc attcccatca agaacacctc cgcctggcag tgcagatgca 2700
aaagcccttc ccaatccagg ggattaccac tggaggaaaa atggtgaagt ccatctaaat 2760
gatcctcttg caatggcaaa attacaagaa gcagctaaag taaacagccg ggaagcatac 2820
aaagaatatt ctaagcgaat tcaagagctt aacaaggcgt gcaatctgcg tggtatgctg 2880
aaatttatag atagcactag caagatttct ttggatgagg ttgaacctgc aagtgagata 2940
gtgaaacgct tttgtactgg ggcaatgagt tatggatcta tttcgttgga ggcacatact 3000
gcccttgctg tggccatgaa caaactggga ggcaaatcta acacagggga gggaggggag 3060
cagccttctc gtatggagcc tcttcctgat ggttcgatga atccaaaacg aagtgcaatc 3120
aagcaagttg ccagtggacg atttggagtt tccagctatt atctgactaa tgcagatggg 3180

```
ctgcagataa aaatggctca gggtgctaag cctggtgaag gtggtgagct tccaggtcac   3240
aaggttatcg gtgacattgc agttaccaga cattctacag ctggtgttgg gcttattagt   3300
ccacctcctc accatgatat atattctatc gaggatcttg cacagcttat ccatgacctt   3360
aagaattcaa atcctcaagc tcgaatcagc gtgaagctag tgtctgaggc tggtgttgga   3420
gttgtagcta gcggtgttgt aaaaggacac gcagaccatg ttcttatttc tggccatgac   3480
ggtggcaccg gtgcatcaag atggacaggt atcaagaatg ctggacttcc atgggaacta   3540
ggattggctg agacacatca aactttagtg gcaaatggcc ttcgtggtcg agctgtccta   3600
caaacagatg gccaattaaa aactggtaga gatgttgctg tggcgtgctt acttggtgca   3660
gaggaatttg gtttcagcac tgctccactg atcacacttg gctgcattat gatgcggaag   3720
tgccatacca atacttgccc tgttggtata gccactcaag atccagtgct ccgagaaaaa   3780
tttgctggag aaccagagca tgtcattaac tttttcttca tgcttgctga ggagctaaga   3840
gaaattatgg ctcagcttgg cctccgtaca attaatgaaa tggttggacg ctctgatatg   3900
cttgaagttg atccagaagt agttaagagc aatgagaaac ttgaaaatat tgatctctca   3960
ttgatcttaa aaccagctgc agagattcgt cctggggctg ctcagtactg tgttgaaaag   4020
caagaccatg gccttgacat ggctttggat aacaaactta tagctttatc aagggctgca   4080
cttgaaaagg aagttcgtgt tttcattgag actccaatca agaacactaa tcgggcagta   4140
ggcaccacac ttagccatga agtcacaaaa cgttatcaca tgaagggatt agatcctgga   4200
accattcatg tgaaactcac tggaagtgct ggtcagagtt ttggtgcttt tctctgtcct   4260
ggaataaccc ttgagcttga aggagacagc aatgattacg ttggcaaagg attatctggt   4320
ggaaagattg ttgtgtaccc acccaggaac agtacattta gtgcagaaga caatattgtc   4380
attggtaatg tggccctgta tggtgctacc aagggagaag catacttcaa tggaatggca   4440
gcagaaaggt tttgtgttcg taattctggt gctcgaacag tggttgaagg aattggtgat   4500
catggatgcg agtacatgac aggggggtact gtagtcatcc ttggtaaaac aggaagaaat   4560
tttgctgctg ggatgagtgg aggcatcgct tatgtttatg atgttgatgg gacattcagt   4620
gtccgctgta acaatgagtt ggttgatcta tatcatgtgg aggaagagga tgatgtaacc   4680
actttgaaaa tgatgataga gcaacatcga cttcacacag agagtgtcct ggccaaagac   4740
atactctcta aattcgacac tcttcttcca aaatttgtaa aagtataccc aagggattat   4800
aagagggttc tagaagaaat gaaggcagaa aaagctgcag ctaggcctac aaaggaacct   4860
aaggtggcaa atggtgtttc tgtgacaact aagaaaatac aaacagagaa gtcatccagc   4920
cgaccaacac gtgttgccaa cgccaaaaag taccggggtt ttgtaacata tgagcgagag   4980
ggtgtttctt accgtgatcc aaatgagcgt gttaaagatt ggaacgaggt tgccattgaa   5040
tcagttccag ggccactgtt aaacacacag tctgctcgtt gtatggattg tggcactcct   5100
ttctgtcatc aggaaagctc aggtgctggc tgtcctcttg gaaataagat cccagagttc   5160
aatgaattag ttcaccagaa tagatggcgt gaagcattgg atcgcctact agagacaaac   5220
aacttccctg aatttactgg acgtgtgtgc cctgctcctt gtgaggggtc ttgtgttctt   5280
ggcattattg agaacccagt atcaatcaaa agcatagaat gctcgattat agacaaaggt   5340
tttgaagagg gatggatggt accacgacca ccacttcaaa gaacaggaaa gaaaatcgct   5400
atagttggca gtggtcctgc tggtttggct gccgctgatc aactaaataa aatgggccat   5460
```

tttgtaactg tatttgaacg ttcggatcgt ataggaggtc ttatgatgta tggagtacca 5520
aacatgaaga cagacaagat tggagttgtt cagcgtcgtg tcaatttaat ggccgaagag 5580
ggtgtaacat ttgtggtgaa tgctaatgta gggagtgatc ctttatactc aattgaacgt 5640
ctccattctg agaacaatgc agttattttg gcttgtggag ctacaaaacc aagggacctc 5700
agtattcctg gccgtgagct agctggagtt cattttgcca tggaatttct ccacgcaaat 5760
accaaaagtt tgcttgatag caacctggag gatggaagat acatatctgc ccagggtaag 5820
aaggtggtgg tcattggtgg tggagacaca ggcacagatt gcatcggtac gtctgttagg 5880
catggttgca gcagcattgt aaatctggag cttctcacca agccaccaag caagagagct 5940
tctgacaacc cctggcccca gtggcctaga gtcttccgag tggactatgg gcaccaggaa 6000
gcatctacca aatttggaaa tgatccaaga acttactaag tcttaaccaa gcgtttcatt 6060
ggtgatgaag atggaaaatt gaaggccctt gaggtggtgc gcgtgaagtg ggagaaagta 6120
gatggaaaat tccagttcaa ggagattgaa ggatcacaag agatcatcga ggcagacctt 6180
gtcctccttg ccatgggatt cctgggccct gaagagaaca tcgctgacaa actgggttta 6240
gagaaagaca accgttccaa cttcaaagct caattcggac acttcgggac cagtgtggat 6300
ggcgtttttg ccgctgggga ttgcaggcgc gggcaatcgc tggttgtttg ggccatcacc 6360
gaagggcgtg aagctgctgc tgcagtagat aagtacttgt caagggatga acaaaatgtc 6420
gcaggcctta ca 6432

<210> 6

<211> 2167

<212> PRT

<213> Oryza sativa

<400> 6

Met Ser Ala Ala Gln Gly Met Ala Tyr Lys Leu Arg Thr Asp Ala Ala
1 5 10 15

Pro Thr Gly Ala Gly Arg Arg Ala Arg Arg Ser His Ser Ser Val Ala
20 25 30

Ala Pro Tyr Arg Ala Ala Arg Leu Val Gln Gly Gly Val Ser Ile Glu
35 40 45

Gly Gly Leu Val Gly Gly Cys Gln Leu Thr Glu Glu Arg Val Ala Ala
50 55 60

Arg Pro Pro Arg Ala Ala Ala Arg Asp Ala Glu Pro Val Arg Pro Leu
65 70 75 80

Ser Thr Leu Pro Glu Ser Ser Ile Gly Leu Tyr Asp Pro Ser Arg Glu
85 90 95

Arg Asp Ser Cys Gly Val Gly Phe Val Ala Glu Leu Ser Gly Asp Tyr
100 105 110

Lys Arg Ala Thr Val Asn Asp Ala Leu Glu Met Leu Glu Arg Met Ala
115 120 125

His Arg Gly Ala Cys Gly Cys Glu Lys Asn Thr Gly Asp Gly Ala Gly
130 135 140

Ile Leu Val Ala Leu Pro His Asn Phe Phe Arg Glu Val Thr Lys Asp
145 150 155 160

Ala Gly Phe Glu Leu Pro Gln Pro Gly Glu Tyr Ala Val Gly Met Val
165 170 175

Phe Leu Pro Ile Asp Glu Lys Arg Arg Glu Arg Ser Lys Ala Glu Phe
180 185 190

Gln Lys Val Ala Glu Ser Leu Gly His Val Ile Leu Gly Trp Arg Arg
195 200 205

Val Pro Thr Asp Asn Ser Asp Leu Gly Glu Ser Ala Leu Gln Thr Glu
210 215 220

Pro Val Ile Glu Gln Val Phe Leu Thr Lys Ser Ser Ser Ser Glu Ala
225 230 235 240

Asp Phe Glu Gln Gln Leu Tyr Ile Leu Arg Arg Leu Ser Ile Leu Ser
245 250 255

Ile Arg Ala Ala Leu Asn Leu Arg Arg Gly Gly Lys Arg Asp Phe Tyr
260 265 270

Met Cys Ser Leu Ser Ser Arg Thr Ile Val Tyr Lys Gly Gln Leu Lys
275 280 285

Pro Cys Gln Leu Lys Gly Tyr Tyr Tyr Ala Asp Leu Gly His Glu Asn
290 295 300

Phe Thr Ser Tyr Met Ala Leu Val His Ser Arg Phe Ser Thr Asn Thr
305 310 315 320

Phe Pro Ser Trp Asp Arg Ala Gln Pro Met Arg Val Leu Gly His Asn
325 330 335

Gly Glu Ile Asn Thr Leu Lys Gly Asn Lys Asn Trp Met Lys Ala Arg
340 345 350

Glu Gly Leu Leu Glu Cys Glu Lys Leu Gly Leu Thr Lys Asp Gln Phe
355 360 365

Ser Lys Ile Leu Pro Ile Val Asp Ala Thr Ser Ser Asp Ser Gly Ala
370 375 380

Phe Asp Gly Val Leu Glu Leu Leu Ile Arg Gly Gly Arg Ser Leu Pro
385 390 395 400

Glu Ala Val Met Met Met Ile Pro Glu Ala Trp Gln Asn Asp Val Asn
405 410 415

Met Glu Pro Glu Lys Lys Ala Leu Tyr Glu Phe Leu Ser Ala Leu Met
420 425 430

Glu Pro Trp Asp Gly Pro Ala Leu Ile Ser Phe Thr Asp Gly Arg Tyr
435 440 445

Leu Gly Ala Thr Leu Asp Arg Asn Gly Leu Arg Pro Gly Arg Phe Tyr
450 455 460

Val Thr His Ser Gly Arg Val Val Met Gly Ser Glu Val Gly Val Val
465 470 475 480

Asp Val Pro Ser Lys Asp Val Leu Arg Lys Gly Arg Leu Asn Pro Gly
485 490 495

Met Met Leu Leu Val Asp Phe Glu Asn His Thr Val Val Asp Asp Glu
500 505 510

Ala Leu Lys Ala Gln Tyr Ser Lys Ala His Pro Tyr Gly Glu Trp Leu
515 520 525

Lys Arg Gln Lys Ile Tyr Leu Lys Asp Ile Val Glu Ser Val Pro Glu
530 535 540

Thr Glu Arg Val Ala Pro Gly Ile Ser Gly Ser Leu Thr Gln Lys Asn
545 550 555 560

Glu Lys Lys Glu His Ala Gly Val Asn Gly Ile Val Thr Pro Leu Lys
565 570 575

Ala Phe Gly Tyr Thr Val Glu Ala Leu Glu Met Leu Leu Leu Pro Met
580 585 590

Ala Lys Asp Gly Val Glu Ala Leu Gly Ser Met Gly Asn Asp Thr Pro
595 600 605

Leu Ala Val Met Ser Asn Arg Glu Lys Leu Thr Phe Glu Tyr Phe Lys
610 615 620

Gln Met Phe Ala Gln Val Thr Asn Pro Pro Ile Asp Pro Ile Arg Glu
625 630 635 640

Lys Ile Val Thr Ser Met Glu Cys Met Ile Gly Pro Glu Gly Asp Leu
645 650 655

Leu Glu Thr Thr Glu Lys Gln Cys Asn Arg Leu Ala Leu Glu Gly Pro
660 665 670

Leu Val Ser Ile Asp Glu Met Glu Ala Ile Lys Lys Met Asn Tyr Arg
675 680 685

Gly Trp Arg Ser Lys Val Leu Asp Ile Thr Tyr Pro Lys Lys Ser Gly
690 695 700

Arg Lys Gly Leu Glu Glu Thr Leu Asp Arg Ile Cys Thr Glu Ala Arg
705 710 715 720

Gly Ala Ile Lys Lys Gly Tyr Thr Val Leu Val Leu Ser Asp Arg Gly
725 730 735

```
Phe Ser Ser Asp Arg Val Ala Val Ser Ser Leu Leu Ala Val Gly Ala
            740                 745                 750
Val His Gln His Leu Val Ala Asn Leu Glu Arg Thr Arg Val Gly Leu
        755                 760                 765
Leu Val Glu Ser Ala Glu Pro Arg Glu Val His His Phe Cys Thr Leu
    770                 775                 780
Val Gly Phe Gly Ala Asp Ala Val Cys Pro Tyr Leu Ala Ile Glu Ala
785                 790                 795                 800
Ile Trp Cys Leu Gln Asn Asp Gly Lys Ile Pro Pro Asn Gly Asp Gly
                805                 810                 815
Lys Pro Tyr Ser Lys Glu Glu Leu Val Lys Lys Tyr Phe Tyr Ala Ser
            820                 825                 830
Asn Tyr Gly Met Met Lys Val Leu Ala Lys Met Gly Ile Ser Thr Leu
        835                 840                 845
Ala Ser Tyr Lys Gly Ala Gln Ile Phe Glu Ala Leu Gly Leu Ser Ser
    850                 855                 860
Glu Val Ile Arg Lys Cys Phe Asp Gly Thr Pro Ser Arg Ile Glu Gly
865                 870                 875                 880
Ala Thr Phe Glu Met Leu Ala Arg Asp Ala Leu Arg Leu His Glu Leu
                885                 890                 895
Ala Phe Pro Ser Arg Ala Pro Pro Pro Gly Ser Ala Asp Ala Lys Ala
            900                 905                 910
Leu Pro Asn Pro Gly Asp Tyr His Trp Arg Lys Asn Gly Glu Val His
        915                 920                 925
Leu Asn Asp Pro Leu Ala Met Ala Lys Leu Gln Glu Ala Ala Arg Val
    930                 935                 940
Asn Ser Arg Ala Ala Tyr Lys Glu Tyr Ser Arg Arg Ile Gln Glu Leu
945                 950                 955                 960
Asn Lys Thr Cys Asn Leu Arg Gly Met Leu Lys Phe Lys Asp Thr Ala
                965                 970                 975
Asp Met Ile Ser Val Asp Glu Val Glu Pro Ala Ser Glu Ile Val Lys
            980                 985                 990
Arg Phe Val Thr Gly Ala Met Ser  Tyr Gly Ser Ile Ser  Leu Glu Ala
        995                 1000                1005
His Thr  Ala Leu Ala Met Ala  Met Asn Lys Leu Gly  Gly Lys Ser
    1010                1015                1020
Asn Thr  Gly Glu Gly Gly Glu  Gln Pro Ser Arg Met  Glu Pro Leu
    1025                1030                1035
```

Ala Asn Gly Ser Met Asn Pro Lys Arg Ser Ala Ile Lys Gln Val
1040 1045 1050
Ala Ser Gly Arg Phe Gly Val Ser Ser Tyr Tyr Leu Thr Asn Ala
1055 1060 1065
Asp Glu Leu Gln Ile Lys Met Ala Gln Gly Ala Lys Pro Gly Glu
1070 1075 1080
Gly Gly Glu Leu Pro Gly His Lys Val Ile Gly Asp Ile Ala Val
1085 1090 1095
Thr Arg His Ser Thr Ala Gly Val Gly Leu Ile Ser Pro Pro Pro
1100 1105 1110
His His Asp Ile Tyr Ser Ile Glu Asp Leu Ala Gln Leu Ile His
1115 1120 1125
Asp Leu Lys Asn Ser Asn Pro Arg Ala Arg Ile Ser Val Lys Leu
1130 1135 1140
Val Ser Glu Ala Gly Val Gly Val Val Ala Ser Gly Val Val Lys
1145 1150 1155
Gly His Ala Asp His Val Leu Ile Ser Gly His Asp Gly Gly Thr
1160 1165 1170
Gly Ala Ser Arg Trp Thr Gly Ile Lys Asn Ala Gly Leu Pro Trp
1175 1180 1185
Glu Leu Gly Leu Ala Glu Thr His Gln Thr Leu Val Ala Asn Gly
1190 1195 1200
Leu Arg Gly Arg Ala Ile Leu Gln Thr Asp Gly Gln Leu Lys Thr
1205 1210 1215
Gly Lys Asp Val Ala Val Ala Cys Leu Leu Gly Ala Glu Glu Phe
1220 1225 1230
Gly Phe Ser Thr Ala Pro Leu Ile Thr Leu Gly Cys Ile Met Met
1235 1240 1245
Arg Lys Cys His Thr Asn Thr Cys Pro Val Gly Ile Ala Thr Gln
1250 1255 1260
Asp Pro Val Leu Arg Glu Lys Phe Ala Gly Glu Pro Glu His Val
1265 1270 1275
Ile Asn Phe Phe Phe Met Leu Ala Glu Glu Leu Arg Glu Ile Met
1280 1285 1290
Ser Gln Leu Gly Phe Arg Thr Ile Thr Glu Met Val Gly Arg Ser
1295 1300 1305
Asp Met Leu Glu Val Asp Pro Glu Val Val Lys Ser Asn Glu Lys
1310 1315 1320

Leu Glu Asn Ile Asp Leu Ser Leu Ile Leu Lys Pro Ala Ala Glu
1325 1330 1335

Ile Arg Pro Gly Ala Ala Gln Tyr Cys Val Glu Lys Gln Asp His
1340 1345 1350

Gly Leu Asp Met Ala Leu Asp Asn Lys Leu Ile Ala Leu Ser Lys
1355 1360 1365

Ala Ala Leu Glu Lys Glu Val Arg Val Phe Ile Glu Thr Pro Ile
1370 1375 1380

Gln Asn Thr Asn Arg Ala Val Gly Thr Met Leu Ser His Glu Val
1385 1390 1395

Thr Lys Arg Tyr His Met Lys Gly Leu Pro Ala Gly Thr Ile His
1400 1405 1410

Val Lys Leu Thr Gly Ser Ala Gly Gln Ser Leu Gly Ala Phe Leu
1415 1420 1425

Cys Pro Gly Ile Thr Leu Glu Leu Glu Gly Asp Ser Asn Asp Tyr
1430 1435 1440

Val Gly Lys Gly Leu Ser Gly Gly Lys Ile Val Val Tyr Pro Pro
1445 1450 1455

Arg Asp Ser Thr Phe Ile Pro Glu Asp Asn Ile Val Ile Gly Asn
1460 1465 1470

Val Ala Leu Tyr Gly Ala Thr Ile Gly Glu Ala Tyr Phe Asn Gly
1475 1480 1485

Met Ala Ala Glu Arg Phe Cys Val Arg Asn Ser Gly Ala Gln Ala
1490 1495 1500

Val Val Glu Gly Ile Gly Asp His Gly Cys Glu Tyr Met Thr Gly
1505 1510 1515

Gly Thr Val Val Ile Leu Gly Lys Thr Gly Arg Asn Phe Ala Ala
1520 1525 1530

Gly Met Ser Gly Gly Ile Ala Tyr Val Tyr Asp Ile Asp Gly Lys
1535 1540 1545

Phe Ser Val Arg Cys Asn His Glu Leu Val Asp Leu Tyr His Val
1550 1555 1560

Glu Glu Glu Glu Asp Ile Thr Thr Leu Lys Met Met Ile Glu Gln
1565 1570 1575

His Arg Leu Asn Thr Gly Ser Val Val Ala Arg Asp Ile Leu Ser
1580 1585 1590

Asn Phe Asp Thr Leu Leu Pro Lys Phe Val Lys Val Phe Pro Arg
1595 1600 1605

Asp Tyr Lys Arg Val Leu Asp Asn Met Lys Ala Glu Lys Ala Ala
1610 1615 1620

Ala Lys Leu Ala Lys Glu Pro Lys Ile Ser Asn Gly Val Ser Val
1625 1630 1635

Thr Thr Lys Lys Val Gln Pro Glu Gln Ser Thr Asn Arg Pro Thr
1640 1645 1650

Arg Val Ser Asn Ala Lys Lys Tyr Arg Gly Phe Ile Ser Tyr Glu
1655 1660 1665

Arg Glu Ser Ile Ser Tyr Arg Asp Pro Asn Glu Arg Val Lys Asp
1670 1675 1680

Trp Lys Glu Val Ala Ile Glu Ser Val Pro Gly Pro Leu Leu Asn
1685 1690 1695

Thr Gln Ser Ala Arg Cys Met Asp Cys Gly Thr Pro Phe Cys His
1700 1705 1710

Gln Glu Ser Ser Gly Ala Gly Cys Pro Leu Gly Asn Lys Ile Pro
1715 1720 1725

Glu Phe Asn Glu Leu Val His Gln Asn Arg Trp Arg Glu Ala Leu
1730 1735 1740

Asp Arg Leu Leu Glu Thr Asn Asn Phe Pro Glu Phe Thr Gly Arg
1745 1750 1755

Val Cys Pro Ala Pro Cys Glu Gly Ser Cys Val Leu Gly Ile Ile
1760 1765 1770

Glu Asn Pro Val Ser Ile Lys Ser Ile Glu Cys Ala Ile Ile Asp
1775 1780 1785

Lys Gly Phe Glu Glu Gly Trp Met Val Pro Arg Pro Pro Leu Gln
1790 1795 1800

Arg Thr Gly Lys Lys Val Ala Ile Ile Gly Ser Gly Pro Ala Gly
1805 1810 1815

Leu Ala Ala Ala Asp Gln Leu Asn Lys Met Gly His Phe Val Thr
1820 1825 1830

Val Phe Glu Arg Ala Asp Arg Ile Gly Gly Leu Met Met Tyr Gly
1835 1840 1845

Val Pro Asn Met Lys Thr Asp Lys Ile Glu Ile Val Gln Arg Arg
1850 1855 1860

Val Asn Leu Met Ala Glu Glu Gly Ile Thr Phe Val Val Asn Ala
1865 1870 1875

Asn Val Gly Ser Asp Pro Leu Tyr Ser Ile Glu Arg Leu Arg Ser
1880 1885 1890

Glu Asn Asp Ala Val Ile Leu Ala Cys Gly Ala Thr Lys Pro Arg
1895 1900 1905

Asp Leu Gly Ile Pro Gly Arg Glu Leu Ser Gly Val His Phe Ala
1910 1915 1920

Met Glu Phe Leu His Ala Asn Thr Lys Ser Leu Leu Asp Ser Asn
1925 1930 1935

Leu Glu Asp Gly Arg Tyr Ile Ser Ala Lys Gly Lys Lys Val Val
1940 1945 1950

Val Ile Gly Gly Gly Asp Thr Gly Thr Asp Cys Ile Gly Thr Ser
1955 1960 1965

Ile Arg His Gly Cys Thr Ser Ile Val Asn Leu Glu Leu Leu Thr
1970 1975 1980

Lys Pro Pro Ser Lys Arg Ala Ala Asp Asn Pro Trp Pro Gln Trp
1985 1990 1995

Pro Arg Ile Phe Arg Val Asp Tyr Gly His Gln Glu Ala Ser Ser
2000 2005 2010

Lys Phe Gly Asn Asp Pro Arg Thr Tyr Glu Val Leu Thr Lys Arg
2015 2020 2025

Phe Ile Gly Asp Glu Asn Gly Asn Val Lys Ala Leu Glu Val Val
2030 2035 2040

Arg Val Lys Trp Glu Lys Val Asp Gly Arg Phe Gln Phe Lys Glu
2045 2050 2055

Ile Glu Gly Ser Asn Glu Thr Ile Glu Ala Asp Leu Val Leu Leu
2060 2065 2070

Ala Met Gly Phe Leu Gly Pro Glu Ala Thr Ile Ala Glu Lys Leu
2075 2080 2085

Gly Leu Glu Lys Asp Asn Arg Ser Asn Phe Lys Ala Gln Phe Gly
2090 2095 2100

Asn Phe Ala Thr Ser Val Asp Gly Ile Phe Ala Ala Gly Asp Cys
2105 2110 2115

Arg Arg Gly Gln Ser Leu Val Val Trp Ala Ile Thr Glu Gly Arg
2120 2125 2130

Gln Ala Ala Ala Ala Val Asp Lys Tyr Leu Ser Arg Asn Glu Gln
2135 2140 2145

Asp Ala Ala Glu Asp Ile Thr Pro Ser Gly Ala Gly Phe Val Gln
2150 2155 2160

Pro Val Ala Ala
2165

<210> 7

<211> 20

<212> DNA

<213> Artificial

<220>

<223> Primer

<400> 7

ttagtggcaa atgggcttcg 20

<210> 8

<211> 21

<212> DNA

<213> Artificial

<220>

<223> Primer

<400> 8

cgccacagca acatctctac c 21

<210> 9

<211> 21

<212> DNA

<213> Artificial

<220>

<223> Primer

<400> 9

cagctgcaga gattcgtcct g 21

<210> 10

<211> 23

<212> DNA

<213> Artificial

<220>

<223> Primer

<400> 10

tgttatccaa agccatgtca agg 23

<210> 11

<211> 20

<212> DNA

<213> Artificial

<220>

<223> Primer

<400> 11
tggaatggca gcagaaaggt 20

<210> 12
<211> 21
<212> DNA
<213> Artificial
<220>
<223> Primer
<400> 12
tcgcatccat gatcaccaat t 21

<210> 13
<211> 23
<212> DNA
<213> Artificial
<220>
<223> Primer
<400> 13
gcaccatttc tgtacactcg ttg 23

<210> 14
<211> 22
<212> DNA
<213> Artificial
<220>
<223> Primer
<400> 14
atcttcccat cagtctgcaa gc 22

<210> 15
<211> 22
<212> DNA
<213> Artificial
<220>
<223> Primer
<400> 15
ttcaagagct taacaaggcg tg 22

<210> 16
<211> 22
<212> DNA
<213> Artificial

<220>

<223> Primer

<400> 16

cacttgcagg ttcaacctca tc 22

<210> 17

<211> 20

<212> DNA

<213> Artificial

<220>

<223> Primer

<400> 17

tgggaaatga tgcacccta 20

<210> 18

<211> 23

<212> DNA

<213> Artificial

<220>

<223> Primer

<400> 18

cttgtgcaaa catctgcttg aag 23

<210> 19

<211> 21

<212> DNA

<213> Artificial

<220>

<223> Primer

<400> 19

aattctggaa ggaagggctt g 21

<210> 20

<211> 23

<212> DNA

<213> Artificial

<220>

<223> Primer

<400> 20

tttgtatccc tcgcgtatag ctt 23

<210> 21

<211> 500

```
<212>  DNA
<213>  Artificial
<220>
<223>  NADH-GoGAT fragment
<400>  21
ggggtgcagc catgccgacg gcgcagggga ttggtttgaa gcacgcgacg ccgacgggcg     60
tcggccgcag ggcccggcgc agccacgccg cgtccgcgca tggccgctcc gcgaggcagg    120
cgcacggtgc catgtctctg gagggcggcg ggttcctcgg cggcgcgcac cgcatcgagg    180
aacgcgtcgc gccatgcccg cctcgggccg cggcgcgtga cgcagagtcg atacggccca    240
tgtctctgct acccgagagc agcattgggc tctacaaccc ggcgttcgag cgtgactcgt    300
gcggcgttgg tttcgtcgcc gagctgtcgg gcgttgacaa ccgggcgacc gtcgtcgatg    360
ccattcagat gcttgaaaga atggcacacc gaggtgcctg cggctgtgag aaaaacactg    420
gtgatggtgc cggcattctc gttgctctac cacacaactt cttccgagag gtgacaaagg    480
atgccggttt cgagttaccg                                                500
```